# EUROPÄISCHE PATENTSCHRIFT

(11) **EP 0 410 233 B1**
(45) Veröffentlichungstag und Bekanntmachung des Hinweises auf die Patenterteilung: **15.11.1995**
(21) Anmeldenummer: 90113459.3
(22) Anmeldetag: 13.07.1990
(51) Int. Cl.: C07C 43/21, C09K 19/30, C09K 19/32, C09K 19/34, C07C 13/28, C07C 69/75, C07C 69/773, C07C 255/50, C07D 319/06, C07D 213/24, C07D 239/26

(54) **(4-Arylbutyl)cyclohexan-Derivate**
(4-Arylbutyl) cyclohexane derivatives
Dérivés d'(aryl-4 butyl) cyclohexane

(30) Priorität: 25.07.1989 CH 2779/89; 10.04.1990 CH 1239/90
(43) Veröffentlichungstag der Anmeldung: 30.01.1991
(73) Patentinhaber: F. HOFFMANN-LA ROCHE AG, 4002 Basel (CH)
(72) Erfinder: Kelly, Stephen, Dr., CH-4313 Möhlin (CH); Leenhouts, Frans, Dr., B-3590 Achel (BE)
(74) Vertreter: Cottong, Norbert A.

(56) Entgegenhaltungen:
- EP-A- 0 104 327
- EP-A- 0 344 557

## Beschreibung

Die vorliegende Erfindung betrifft neue (4-Arylbutyl)cyclohexan-Derivate, flüssigkristalline Gemische, die solche Verbindungen enthalten sowie die Verwendung für elektro-optische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Beispiele solcher Vorrichtungen sind Zellen mit dynamischer Streuung, DAP-Zellen (Deformation aufgerichteter Phasen), Gast/Wirt-Zellen, TN-Zellen ("twisted nematic") und STN-Zellen ("super-twisted nematic") mit verdrillt nematischer Struktur, SBE-Zellen ("super-birefringence effect"), Phase-Change-Zellen mit einem cholesterisch-nematischen Phasenübergang und OMI-Zellen ("optical mode interference"). Die gebräuchlichsten Anzeigevorrichtungen beruhen auf dem Schadt-Helfrich-Effekt und besitzen eine verdrillt nematische Struktur.

In Appl. Phys. Lett. 36, 899 (1980) und in Recent Developments in Condensed Matter Physics 4, 309 (1981) werden ferner elektro-optische Vorrichtungen auf der Basis von chiral getilteten smektischen Flüssigkristallen vorgeschlagen. Hierbei werden die ferroelektrischen Eigenschaften dieser Materialien ausgenutzt. Als getiltete smektische Phasen eignen sich beispielsweise smektisch C, F, G, H, I und K Phasen. Bevorzugt sind im allgemeinen smektisch C Phasen, welche besonders grosse Ansprechgeschwindigkeiten ermöglichen. Die chiral getilteten Phasen werden üblicherweise mit S_{C}* S_{F}* etc. bezeichnet, wobei der Stern die Chiralität angibt.

Die Flüssigkristallmaterialien müssen eine gute chemische und thermische Stabilität und eine hohe Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben. Weiterhin sollen sie bei üblichen Betriebstemperaturen eine geeignete Mesophase besitzen, beispielsweise eine nematische, cholesterische oder chiral getiltete smektische Phase. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine möglichst geringe elektrische Leitfähigkeit aufweisen. Neben dem generellen Interesse an Flüssigkristallmaterialien mit hoher optischer Anisotropie besteht in letzter Zeit ein vermehrtes Interesse an Materialien mit niedriger optischer Anisotropie, insbesondere für aktiv adressierte Flüssigkristallanzeigen, z.B. bei TFT-Anwendungen (Dünnfilmtransistor) in Fernsehgeräten. Anderseits sollten chiral getiltete smektische Flüssigkristalle eine ausreichend hohe spontane Polarisation besitzen.

Flüssigkristalle werden zwecks Optimierung der Eigenschaften in der Regel als Mischungen mehrerer Komponenten verwendet. Es ist daher wichtig, dass die Komponenten untereinander gut mischbar sind. Cholesterische Gemische können vorzugsweise aus einem oder mehreren optisch aktiven Dotierstoffen und einem nematischen Flüssigkristallmaterial bestehen, und ferroelektrische Flüssigkristalle können vorzugsweise aus einem oder mehreren optisch aktiven Dotierstoffen und einem Flüssigkristallmaterial mit getilteter smektischer Phase bestehen.

DE-A-26 17 593 und DE-A-32 25 290 offenbaren Flüssigkristallkomponenten, in den zwei oder mehrere Ringe über eine Brückengruppe mit mehr als 2 Kettenatomen, beispielsweise über eine Gruppe -(CH₂)₄- verknüpft sein können. Enantiotrope Mesophasen wurden jedoch nur für tetracyclische Verbindungen (sowie für gewisse tricyclische Verbindungen, welche aber eine Estergruppe in der Brückengruppe aufweisen) gefunden und die Klärpunkte liegen deutlich tiefer als für entsprechende Verbindungen mit 2 Kettenatomen in der Brückengruppe oder mit direkt verknüpften Ringen.

Gegenstand der vorliegenden Erfindung sind die Verbindungen der allgemeinen Formel 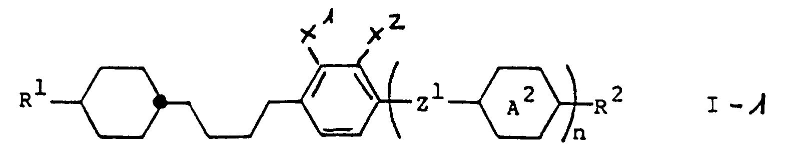
worin n für die Zahl O oder 1 steht; R¹ eine Gruppe R³ oder R³-A³-Z²- und R² eine Gruppe R⁴ oder R⁴-A⁴-Z³- bezeichnen; X¹ und X² unabhängig voneinander Wasserstoff oder Fluor bedeuten; A³, A⁴ trans-1,4-Cyclohexylen bedeuten; und Ring A² unsubstituiertes oder mit Fluor mono- oder disubstituiertes 1,4-Phenylen oder trans-1,4-Cyclohexylen, trans-1,3-Dioxan-2,5-diyl, 1-Cyano-trans-1,4-cyclohexylen, Bicyclo[2.2.2]octan-1,4-diyl, Naphthalin-2,6-diyl, Tetralin-2,6-diyl, trans-Decalin-2,6-diyl Pyridin-2,5-diyl, Pyrimidin-2,5-diyl oder Pyrazin-2,5-diyl darstellt; Z¹, Z² und Z³ je eine einfache Kovalenzbindung bedeuten, oder eine der Gruppen Z¹, Z² und Z³ auch -COO-, -OOC-, -CH₂O-, -OCH₂-, -CH₂CH₂-, -C≡C-, -(CH₂)₃O-, -O(CH₂)₃-, -(CH₂)₄- oder die trans-Form von -CH=CH-CH₂O- oder -OCH₂-CH=CH- bedeutet; R³ und R⁴ unabhängig voneinander Halogen, Cyano, -NCS, -CF₃, -OCF₃ oder Alkyl bezeichnen, in welchem gegebenenfalls eine >CH-CH< durch >C=C< ersetzt ist und/oder gegebenenfalls eine oder zwei nicht benachbarte Methylengruppen durch -O-, -COO- und/oder -OOC-ersetzt sind und/oder gegebenenfalls eine Methylengruppe durch -CHX- ersetzt ist; und X Halogen, Cyano oder Methyl bedeutet.

Es wurde überraschenderweise gefunden, dass die Verbindungen der Formel I-1, welche eine Butan-Brückengruppe zwischen einem Cyclohexanring und dem aromatischen Ring aufweisen, trotz der hohen Flexibilität der Brückengruppe eine ausgeprägte Tendenz zur Ausbildung von flüssigkristallinen Phasen besitzen. Die optisch inaktiven Verbindungen der Formel I-1 besitzen meist eine nematische, eine smektisch A und/oder eine getiltete smektische (vor allem S_{C}) Phase, und die optisch aktiven Verbindungen der Formel I-1 besitzen meist eine cholesterische, eine smektisch A und/oder eine chiral getiltete smektische (vor allem S_{C}*) Phase. Diese Mesophasentypen sind besonders geeignet zur Erziel ung von nematischen, cholesterischen oder chiral getilteten smektischen Phasen in Flüssigkristallgemischen. Verbindungen der Formel I-1, welche eine höher geordnete smektische Phase, z.B. eine smektisch B Phase aufweisen, sind jedoch ebenfalls gut mischbar mit üblichen Flüssigkristallmaterialien. Die vorliegende Erfindung bietet somit eine breite Palette neuer Komponenten und Mischungen zur weiteren Optimierung und Modifizierung von Flüssigkristallmaterialien.

Die Verbindungen der Formel I-1 besitzen eine hohe chemische Stabilität und eine hohe Stabilität gegenüber elektrischen und magnetischen Feldern. Sie sind farblos, leicht herstellbar und gut löslich untereinander und in bekannten Flüssigkristallmaterialien. Ferner besitzen sie tiefe Viskositäten und ergeben in Anzeigevorrichtungen kurze Ansprechzeiten.

Die Eigenschaften der Verbindungen der Formel I-1 können je nach Zahl und Bedeutung der Ringe und der Substituenten in breiten Bereichen variiert werden. Beispielsweise führen aromatische Ringe zu höheren und gesättigte Ringe zu tieferen Werten der optischen Anisotropie. Eine Erhöhung des Klärpunktes kann beispielsweise durch Einführung eines oder mehrerer weiterer Ringe erreicht werden. Polare Endgruppen, wie Cyano, Halogen, -NCS, -CF₃ oder -OCF₃, und Ringe wie Pyrimidin-2,5-diyl, trans-1,3-Dioxan-2,5-diyl etc. erhöhen die dielektrische Anisotropie, und laterale Fluorsubstituenten ergeben eine Beitrag zur Dielektrizitätskonstante sowohl parallel als auch senkrecht zur Moleküllängsachse, was je nach Substitutionsmuster zur Erhöhung oder Verminderung der dielektrischen Anisotropie ausgenützt werden kann. Ferner können durch laterale Substituenten an einem oder mehreren Ringen die Mesophasebereiche modifiziert, eine allfällige Tendenz zur Ausbildung hochgeordneter smektischer Phasen weitgehend unterdrückt und oft auch die Löslichkeit verbessert werden. Weiterhin können durch eine C=C-Doppelbindung in der Seitenkette die elastischen Eigenschaften, die Schwellenspannungen, die Ansprechzeiten, die Mesophasen etc. weiter modifiziert werden.

Die erfindungsgemässen Verbindungen ermöglichen daher eine weitere Optimierung der Flüssigkristallgemische und eine Modifizierung der elektro-optischen Eigenschaften in einem weiten Bereich je nach gewünschten Eigenschaften.

Der Ausdruck "Halogen" bezeichnet im Rahmen der vorliegenden Erfindung Fluor, Chlor, Brom oder Jod, insbesondere Fluor oder Chlor.

Der Ausdruck "unsubstituiertes oder mit Fluor mono- oder disubstituiertes 1,4-Phenylen" umfasst Gruppen wie 1,4-Phenylen, 2-Fluor-1,4-phenylen, 2,3-Difluor-1,4-phenylen.

Der Ausdruck "Tetralin-2,6-diyl" bezeichnet 1,2,3,4-Tetrahydronaphthalin-2,6-diyl. Der Ausdruck "trans-Decalin-2,6-diyl" umfasst von trans-Decahydronaphthalin abgeleitete, 2,6-disubstituierte Gruppen, insbesondere (4aαH,8aβH)-Decahydronaphthalin-2α,6β-diyl.

Der Ausdruck "Alkyl, in welchem gegebenenfalls eine >CH-CH< durch >C=C< ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch -CHX- ersetzt ist," umfasst geradkettige und verzweigte (gegebenenfalls chirale) Reste wie Alkyl, 1E-Alkenyl, 3E-Alkenyl, 4-Alkenyl, Alkenyl mit endständiger Doppelbindung, Alkoxy, 2E-Alkenyloxy, 3-Alkenyloxy, Alkenyloxy mit endständiger Doppelbindung, Alkoxyalkyl, Alkenyloxyalkyl, Alkoxycarbonyl, Alkoxycarbonylalkoxy, Alkanoyloxy, 1-Haloalkyl, 2-Haloalkyl, 2-Haloalkoxy, 2-Haloalkoxycarbonyl, 1-Cyanoalkyl, 2-Cyanoalkyl, 2-Cyanoalkoxy, 2-Cyanoalkoxycarbonyl, 1-Methylalkyl, 2-Methylalkyl, 1-Methylalkoxy, 2-Methylalkoxy, 2-Methylalkoxycarbonyl und dergleichen. Beispiele bevorzugter Reste sind Methyl, Aethyl, Propyl, Butyl, Pentyl, Hexyl, Heptyl, Octyl, Nonyl, Decyl, Undecyl, Dodecyl, 1-Methylpropyl, 1-Methylheptyl, 2-Methylbutyl, 3-Methylpentyl, Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4Z-Heptenyl, 5-Hexenyl, 6-Heptenyl, 7-Octenyl, 8-Nonenyl, 9-Decenyl, 10-Undecenyl, 11-Dodecenyl, Methoxy, Aethoxy, Propyloxy, Butyloxy, Pentyloxy, Hexyloxy, Heptyloxy, Octyloxy, Nonyloxy, Decyloxy, Undecyloxy, Dodecyloxy, 1-Methylpropyloxy, 1-Methylheptyloxy, 2-Methylbutyloxy, 3-Methylpentyloxy, Allyloxy, 2E-Butenyloxy, 2E-Pentenyloxy, 2E-Hexenyloxy, 2E-Heptenyloxy, 3-Butenyloxy, 3Z-Pentenyloxy, 3Z-Hexenyloxy, 3Z-Heptenyloxy, 4-Pentenyloxy, 5-Hexenyloxy, 6-Heptenyloxy, 7-Octenyloxy, 8-Nonenyloxy, 9-Decenyloxy, 10-Undecenyloxy, 11-Dodecenyloxy, Methoxymethyl, Aethoxymethyl, Propyloxymethyl, Allyloxymethyl, Methoxycarbonyl, Aethoxycarbonyl, Propyloxycarbonyl, 1-Methylpropyloxycarbonyl, 1-(Methoxycarbonyl)äthoxy, 1-(Aethoxycarbonyl)äthoxy, Acetoxy, Propionyloxy, Butyryloxy, 1-Fluorpropyl, 1-Fluorpentyl, 1-Chlorpropyl, 2-Fluorpropyl, 2-Fluorpentyl, 2-Chlorpropyl, 2-Fluorpropyloxy, 2-Fluorbutyloxy, 2-Fluorpentyloxy, 2-Fluorhexyloxy, 2-Chlorpropyloxy, 2-Fluorbutyloxy, 2-Fluorpropyloxycarbonyl, 2-Fluorbutyloxycarbonyl, 2-Fluorpentyloxycarbonyl, 2-Fluor-3-methylbutyloxycarbonyl, 2-Fluor-4-methylpentyloxycarbonyl, 2-Chlorpropyloxycarbonyl, 1-Cyanopropyl, 1-Cyanopentyl, 2-Cyanopropyl, 2-Cyanopentyl, 2-Cyanopropyloxy, 2-Cyanobutyloxy, 2-Cyanopentyloxy, 2-Cyanohexyloxy, 2-Cyanopropyloxycarbonyl, 2-Cyanobutyloxycarbonyl, 2-Cyano-3-methylbutyloxycarbonyl, 2-Cyano-4-methylpentyloxycarbonyl und dergleichen.

Bevorzugte Verbindungen der Formel I sind die Verbindungen der allgemeinen Formeln 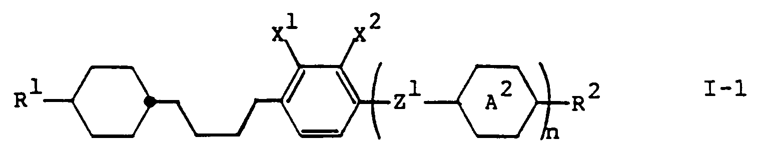
worin n, R¹, R², Z¹ und Ring A² die obigen Bedeutungen haben und X¹ und X² unabhängig voneinander Wasserstoff oder Fluor bezeichnen.

In der obigen Formel I-1 steht Ring A² vorzugsweise für unsubstituiertes oder mit Fluor monosubstituiertes oder 2,3-disubstituiertes 1,4-Phenylen, für trans-1,4-Cyclohexylen, trans-1,3-Dioxan-2,5-diyl, 1-Cyano-trans-1,4-cyclohexylen, Bicyclo-[2.2.2]octan-1,4-diyl, Naphthalin-2,6-diyl, Tetralin-2,6-diyl, trans-Decalin-2,6-diyl oder auch für Pyridin-2,5-diyl, Pyrimidin-2,5-diyl oder Pyrazin-2,5-diyl.

Vorzugsweise stehen Z¹, Z² und Z³ je für eine einfache Konvalenzbindung oder eine der Gruppen Z¹, Z² und Z³ (bevorzugt Z¹) auch für -COO-, -OOC-, -CH₂O-, -OCH₂-, -CH₂CH₂-, -C≡C-, -(CH₂)₃O-, -O(CH₂)₃-, -(CH₂)₄- oder die trans-Form von -CH=CH-CH₂O- oder -OCH₂-CH=CH-.

Bevorzugte Basismaterialien für flüssigkristalline Gemische sind die bicyclischen und die tricyclischen Verbindungen der Formel I-1. In den bicyclischen Verbindungen steht n für die Zahl 0, R¹ für R³ und R² für R⁴. In den tricyclischen Verbindungen steht n für die Zahl 1, R¹ für R³ und R² für R⁴ oder n für die Zahl O, R¹ für R³-A³-Z²- und R² für R⁴ oder n für die Zahl O, R¹ für R³ und R² für R⁴-A⁴-Z³-. Verbindungen mit 4 oder 5 Ringen sind jedoch dann bevorzugt, wenn hohe Klärpunkte erwünscht sind (z.B. zur Verwendung als Dotierstoff zur Klärpunktserhöhung oder als stationäre Phase in der Gaschromatographie) .

Beispiele besonders bevorzugter Untergruppen von Verbindungen der Formel I-1 sind die Verbindungen der allgemeinen Formeln 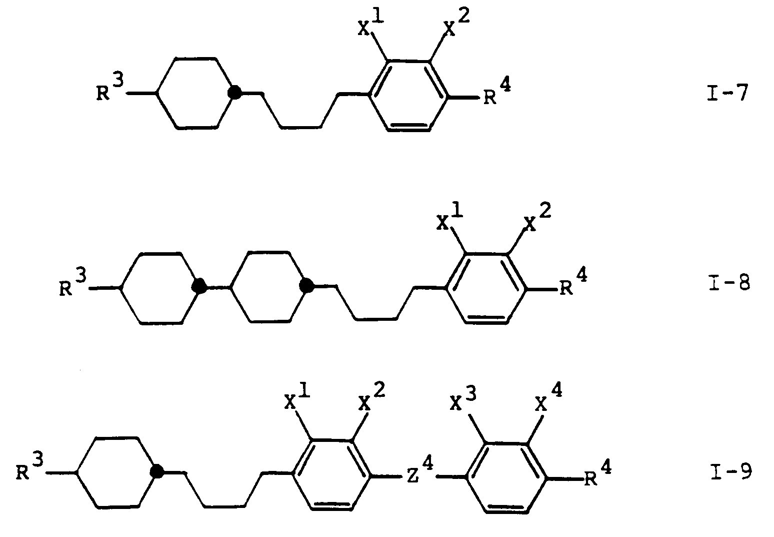 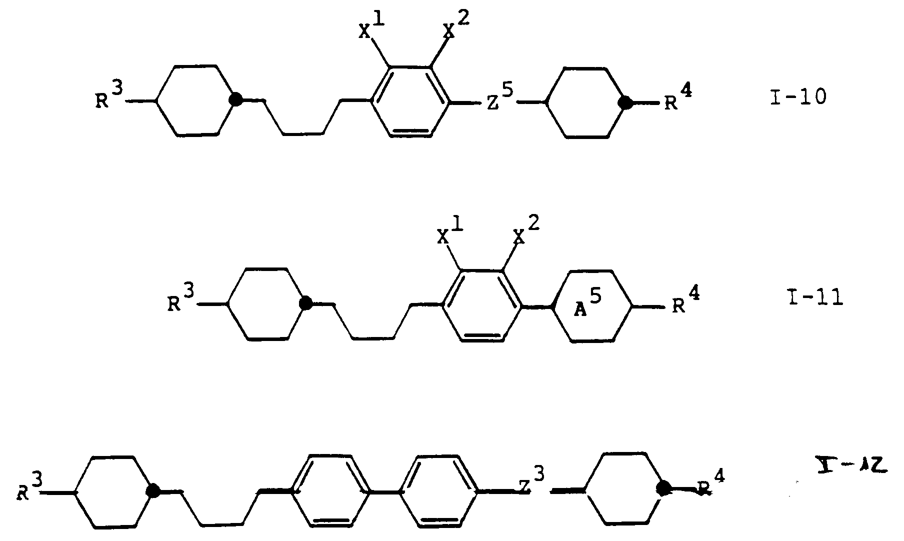
worin R³, R⁴ und Z³ die obigen Bedeutungen haben; X¹, X², X³ und X⁴ unabhängig voneinander Wasserstoff oder Fluor bezeichnen; Z⁴ eine einfache Kovalenzbindung, -COO-, -OOC- oder -C≡C-bedeutet; Z⁵ eine einfache Kovalenzbindung, -OOC-, -OCH₂-, -CH₂CH₂-, -C≡C-, -O(CH₂)₃-, -(CH₂)₄- oder die trans-Form von -OCH₂-CH=CH- bezeichnet; und Ring A⁵ Pyridin-2,5-diyl, Pyrimidin-2,5-diyl, Pyrazin-2,5-diyl, trans-1,3-Dioxan-2,5-diyl, Bicyclo[2.2.2]octan-1,4-diyl, Naphthalin-2,6-diyl, Tetralin-2,6-diyl oder trans-Decalin-2,6-diyl darstellt.

Im allgemeinen sind Verbindungen der Formel I-1 ohne laterale Substituenten oder mit lateralen Fluorsubstituenten (vorzugsweise höchstens einer oder zwei laterale Fluorsubstituenten) bevorzugt, d.h. in obigen Formeln I-1, I-7 bis I-11 stehen die Substituenten X¹, X², X³ und X⁴ vorzugsweise für Wasserstoff oder Fluor, insbesondere für Wasserstoff oder 1 oder 2 der Substituenten X¹, X², X³ und X⁴ auch für Fluor.

In Formel I-11 kann ein gegebenenfalls vorhandener Pyridinring, Pyrimidinring, Pyrazinring oder 1,3-Dioxanring jeweils in 2-Stellung oder in 5-Stellung mit dem Benzolring verknüpft sein.

Vorzugsweise steht höchstens einer der Reste R³ und R⁴ (vorzugsweise R⁴) für Halogen, Cyano, -NCS, -CF₃ oder -OCF₃, d.h. R³ und R⁴ bezeichnen unabhängig voneinander vorzugsweise Alkyl, in welchem gegebenenfalls eine >CH-CH< durch >C=C< ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch -CHX- ersetzt ist, oder einer der Reste R³ und R⁴ (vorzugsweise R⁴) bezeichnet auch Halogen, Cyano, -NCS, -CF₃ oder -OCF₃. Ein endständiger Halogenrest und die Gruppe -NCS stehen vorzugsweise an einem aromatischen Ring, insbesondere einem Benzol-, Pyridin-, Pyrimidin- oder Pyrazinring.

R³ und R⁴ in den obigen Formeln I-1 bis I-12 besitzen vorzugsweise höchstens je etwa 18, insbesondere höchstens je etwa 12 Kohlenstoffatome.

Für nematische und cholesterische Anwendungen sind im allgemeinen kurze Reste R³ und R⁴ (z.B. Reste mit höchstens 12, vorzugsweise höchstens 7 Kohlenstoffatomen) bevorzugt und vorzugsweise kann auch einer der Reste Halogen, Cyano, -NCS, -CF₃ oder -OCF₃ bedeuten. Für smektische Anwendungen (insbesondere getiltete smektische Phasen) sind im allgemeinen diejenigen Verbindungen bevorzugt, worin R³ und R⁴ unabhängig voneinander Alkyl bezeichnen, in welchem gegebenenfalls eine >CH-CH< durch >C=C< ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch -CHX- ersetzt ist, und die Summe der Kohlenstoffatome in R³ und R⁴ zusammen mindestens 10, vorzugsweise mindestens 12 beträgt.

Bevorzugte Reste R³ sind Alkyl, Alkenyl, Alkoxy, Alkenyloxy, Alkoxycarbonyl, Alkenyloxycarbonyl, Alkanoyloxy und Alkenoyloxy, insbesondere Alkyl und Alkenyl. Besonders bevorzugt sind im allgemeinen Reste R³ mit bis zu 12 Kohlenstoffatomen. Bevorzugte Reste R⁴ sind Alkyl, Alkenyl, Alkoxy, Alkenyloxy, Alkoxycarbonyl, Alkenyloxycarbonyl, Alkanoyloxy und Alkenoyloxy (insbesondere Alkyl, Alkenyl, Alkoxy und Alkenyloxy) sowie Halogen (insbesondere Fluor und Chlor), Cyano, -NCS, -CF₃ und -OCF₃. Besonders bevorzugt sind im allgemeinen Reste R⁴ mit bis zu 12 Kohlenstoffatomen.

Geradkettige Reste R³ bzw. R⁴ sind im allgemeinen bevorzugt. Um beispielsweise chirale Dotierstoffe für cholesterische oder für chiral getiltete smektische Flüssigkristalle zu erhalten, können jedoch vorzugsweise auch einer oder beide Reste R³ und R⁴ verzweigt-kettig chiral sein und/oder eine Gruppe -CHX-, worin X Halogen (vorzugsweise Fluor oder Chlor), Cyano oder Methyl bedeutet, anstelle einer Methylengruppe aufweisen. Um für chiral getiltete smektische Anwendungen eine hohe spontane Polarisation zu erhalten, sollte das Chiralitätszentrum (d.h. die Kettenverzweigung oder der Halogen- oder Cyanosubstituent) vorzugsweise nahe am Ringsystem, beispielsweise in 1- oder 2-Stellung des Restes R³ bzw. R⁴ sein. Ferner bleibt die Tendenz zur Bildung flüssigkristalliner Phasen grundsätzlich erhalten, wenn 1 oder 2 nicht benachbarte Methylengruppen durch -O-, -COO- und/oder -OOC- ersetzt werden, was u.a. zur Herstellung chiraler Reste aus natürlichen, optisch aktiven Säuren, Alkoholen etc. ausgenutzt werden kann (z.B. 2-Alkoxycarbonyläthoxy aus Milchsäure).

Die Mesophasenbereiche, die Schwellenspannung, die Ansprechgeschwindigkeit, die Steilheit der Transmissionskennlinie etc. können ferner variiert werden durch Wahl der Stellung der C=C-Doppelbindung in ungesättigten Resten wie Alkenyl, Alkenyloxy und dergleichen. Der Effekt ist grundsätzlich bekannt z.B. aus Mol. Cryst. Liq. Cryst. 122, 241 (1985), 131, 109 (1985) und 148, 123 (1987). Bevorzugt sind Reste, welche die Doppelbindung in 1-Stellung (insbesondere E-Isomer), in 3-Stellung (insbesondere E-Isomer) oder in 4-Stellung (insbesondere Z-Isomer) der Kette unter Einschluss allfälliger Heteroatome aufweisen, wie 1E-Alkenyl, 3E-Alkenyl, 4Z-Alkenyl, 2E-Alkenyloxy, 3Z-Alkenyloxy und dergleichen. Ferner kann die Doppelbindung vorzugsweise auch in endständiger Position stehen, insbesondere im Falle von Verbindungen für smektische Anwendungen. Beispiele bevorzugter Reste mit endständiger Stellung der Doppelbindung sind 6-Heptenyl, 7-Octenyl, 8-Nonenyl, 9-Decenyl, 10-Undecenyl, 11-Dodecenyl, 5-Hexenyloxy, 6-Heptenyloxy, 7-Octenyloxy, 8-Nonenyloxy, 9-Decenyloxy, 10-Undecenyloxy, 11-Dodecenyloxy und dergleichen.

Die Herstellung der Verbindungen der Formel I kann in an sich bekannter Weise erfolgen. Bevorzugte Methoden werden anhand der folgenden Schemata 1-4 veranschaulicht, worin R¹, R², Z¹, n und die Ringe A¹ und A² die obigen Bedeutungen haben, L eine Abgangsgruppe (z.B. Tosylat) darstellt, R C₁-C₁₈-Alkyl bezeichnet und R⁵ eine Alkylgruppe bedeutet, in welcher gegebenenfalls eine >CH-CH< durch >C=C< ersetzt ist und/oder gegebenenfalls eine nicht in 1-Stellung stehenden Methylengruppe durch -O-, -COO- oder -OOC- ersetzt ist und/oder gegebenenfalls eine Methylengruppe durch -CHX- ersetzt (worin X die obige Bedeutung hat). 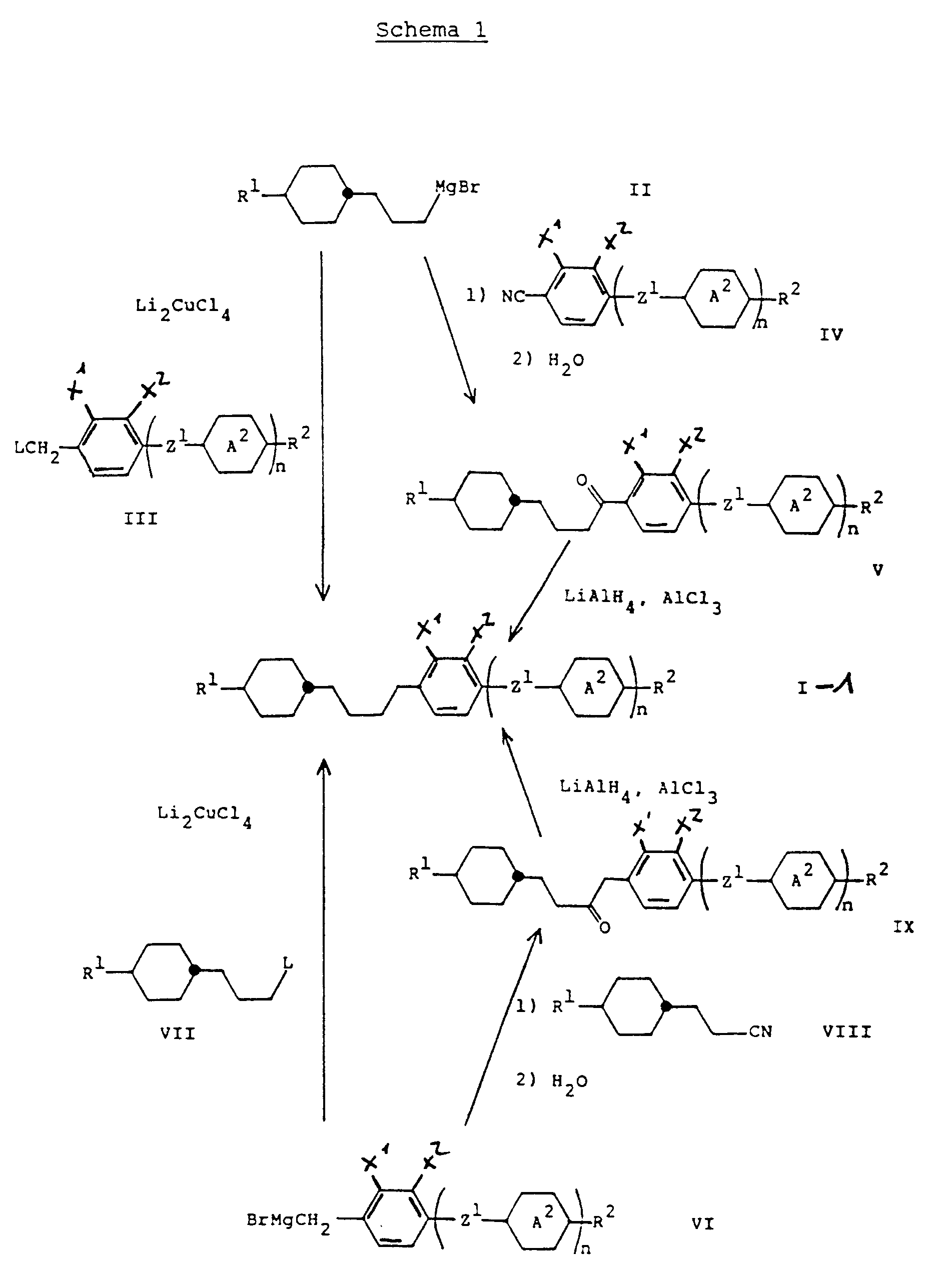 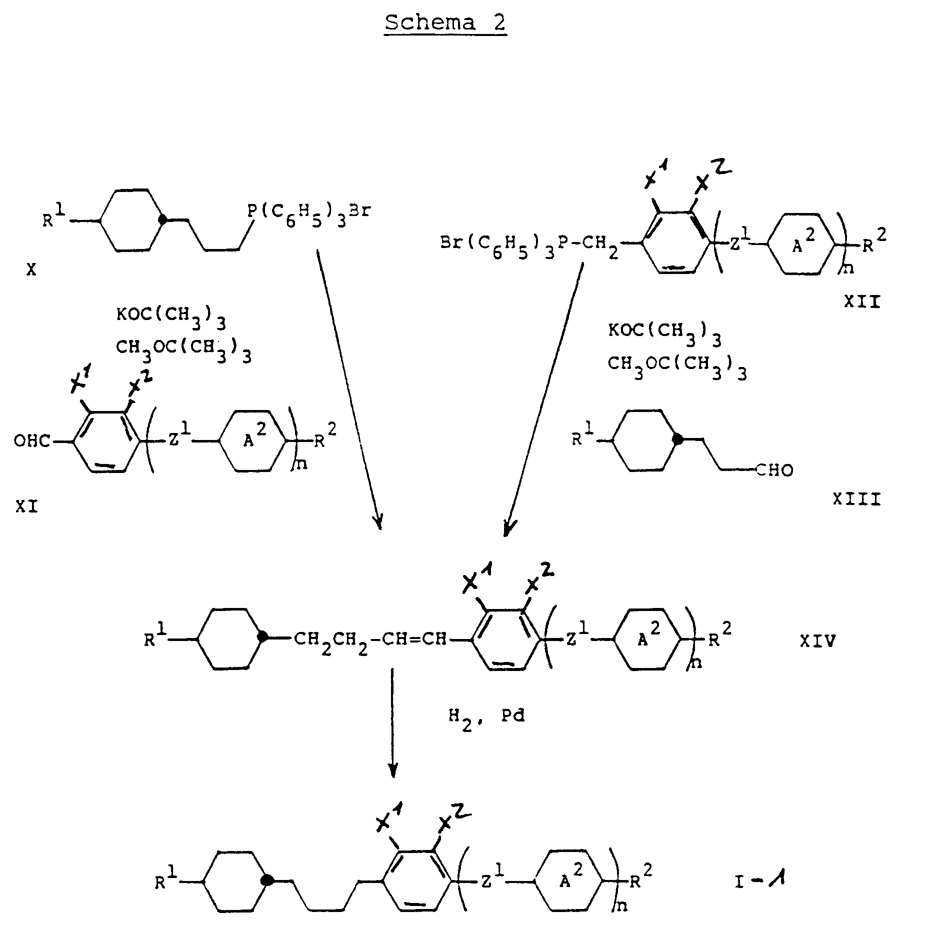 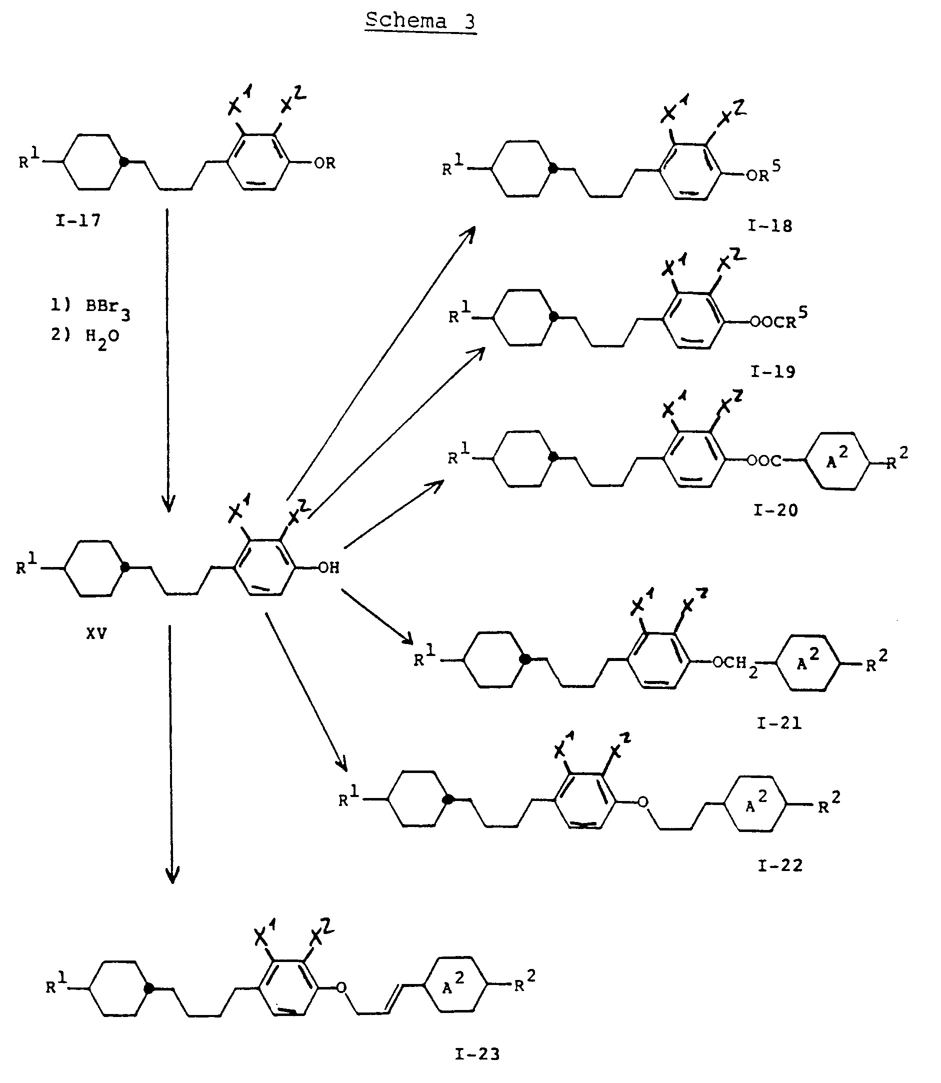 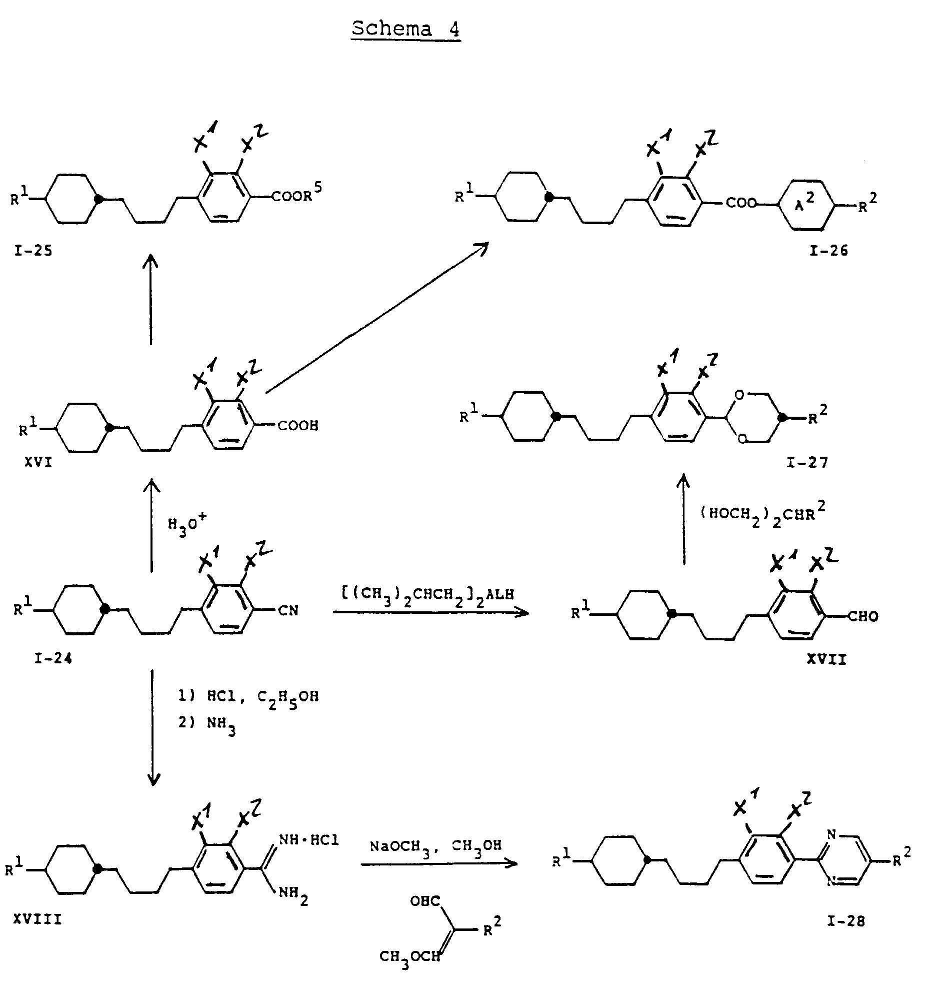
Die Ausgangsmaterialien sind bekannte oder Analoge bekannter Verbindungen und können nach bekannten Methoden hergestellt werden. Insbesondere ist die Herstellung der Grignard-Reagenzien von Schema 1 und der Phosphoniumsalze von Schema 2 aus den entsprechenden Bromiden dem Fachmann geläufig. Die Nitrile der Formeln IV und VIII und die Aldehyde der Formeln XI und XIII sind bekannte Flüssigkristalle, bekannte Zwischenprodukte für Flüssigkristalle oder Analoge solcher Verbindungen.

Analog zu den in den Schemata 1 und 2 dargestellten Reaktionen kann die Verknüpfung auch an anderer Stelle der Butan-Brückengruppe erfolgen.

Bei der Reduktion mit Lithiumaluminiumhydrid und Aluminiumchlorid gemäss Schema 1 besteht die Gefahr, dass Dioxanringe und Estergruppen hydriert werden. Ferner werden bei der katalytischen Hydrierung gemäss Schema 2 auch Doppelbindungen hydriert, welche in den Seitenketten oder in weiteren Brückengruppen (Z¹, Z² oder Z³) vorhanden sein können. Verbindungen der Formel I mit einem Dioxanring oder einer Esterfunktion können jedoch durch Fouquet-Schlosser-Reaktion (z.B. durch Umsetzung einer Verbindung der Formel II mit einer Verbindung der Formel III oder durch Umsetzung einer Verbindung der Formel VI mit einer Verbindung der Formel VII) oder gemäss Schema 2 erhalten werden. Verbindungen mit Doppelbindungen können hingegen nach den in Schema 1 illustrierten Methoden umgesetzt werden.

Vorzugsweise können jedoch Aether- und Esterfunktionen, heterocyclische Ringe, C=C-Doppelbindungen etc. auch erst nach der Synthese der Butan-Brückengruppe gebildet werden. Geeignete Methoden sind dem Fachmann geläufig aus der Herstellung bekannter Flüssigkristallmaterialien. Beispiele solcher Reaktionen sind in den Schemata 3 und 4 illustriert. Weiter Methoden sowie die Einführung von Alkenylsubstituenten und Kettenverlängerungsreaktionen sind z.B. in EP-A-0122389, EP-A-0169327, EP-A-0172360, EP-A-0167912, EP-A-0168683 und EP-A-0242716 offenbart.

Die Verätherung einer Verbindung der Formel XV zu einer Verbindung der Formel I-18, I-21, 1-22 oder I-23 kann in an sich bekannter Weise z.B. durch Umsetzung mit dem entsprechenden Bromid oder Jodid in Gegenwart von Kaliumcarbonat erfolgen. Verbindungen der Formel I-18, welche eine Estergruppe in R⁵ aufweisen, können vorzugsweise auch durch Umsetzung einer Verbindung der Formel XV mit dem entsprechenden Hydroxyester in Gegenwart von Triphenylphosphin und Azodicarbonsäurediäthylester hergestellt werden.

Die Herstellung der Ester der Formeln I-19, I-20, I-25 und I-26 kann nach an sich bekannter Veresterungsmethoden erfolgen, beispielsweise durch Umsetzung einer Verbindung der Formel XV mit der entsprechenden Carbonsäure bzw. durch Umsetzung einer Verbindung der Formel XVI mit dem entsprechenden Alkohol oder Phenol in Gegenwart von 4-(Dimethylamino)pyridin und N,N′-Dicyclohexylcarbodiimid.

Die Verbindungen der Formel I können in Form von Gemischen untereinander und/oder mit andern Flüssigkristallkomponenten verwendet werden. Geeignete Flüssigkristallkomponenten sind dem Fachmann in grosser Zahl bekannt, z.B. aus D. Demus et al., Flüssige Kristalle in Tabellen, VEB Deutscher Verlag für Grundstoffindustrie, Leipzig, Bände I und II, und viele davon sind zudem im Handel erhältlich.

Die Erfindung betrifft daher ebenfalls ein flüssigkristallines Gemisch mit mindestens 2 Komponenten, welches dadurch gekennzeichnet ist, dass mindestens eine Komponente eine Verbindung der Formel I-1 (insbesondere eine der als bevorzugt genannten Verbindungen) ist.

Aufgrund der guten Löslichkeit sowie anderseits der grossen Variationsbreite der Eigenschaften und Anwendungsbereiche, kann der Anteil an Verbindungen der Formel I-1 in den erfindungsgemässen Gemischen in einem breiten Bereich variieren und etwa O,1 bis 1OO Gew.% betragen. Beispielsweise kann das Gemisch aus Verbindungen der Formel I bestehen. Anderseits werden z.B. chirale Dotierstoffe oft nur in relativ kleinen Mengen z.B. etwa O,1 bis 1O Gew.% eingesetzt. Im allgemeinen beträgt jedoch der Anteil an Verbindungen der Formel I-1 in den erfindungsgemässen Gemischen etwa 1 - 60 Gew.%. Bevorzugt ist in der Regel ein Bereich von etwa 5 - 30 Gew.%.

Die erfindungsgemässen Gemische für nematische oder cholesterische Anwendungen enthalten neben einer oder mehreren Verbindungen der Formel I-1 vorzugsweise eine oder mehrere Verbindungen aus der Gruppe der Verbindungen der allgemeinen Formeln 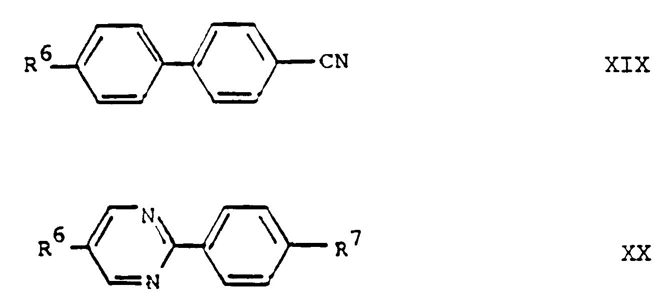 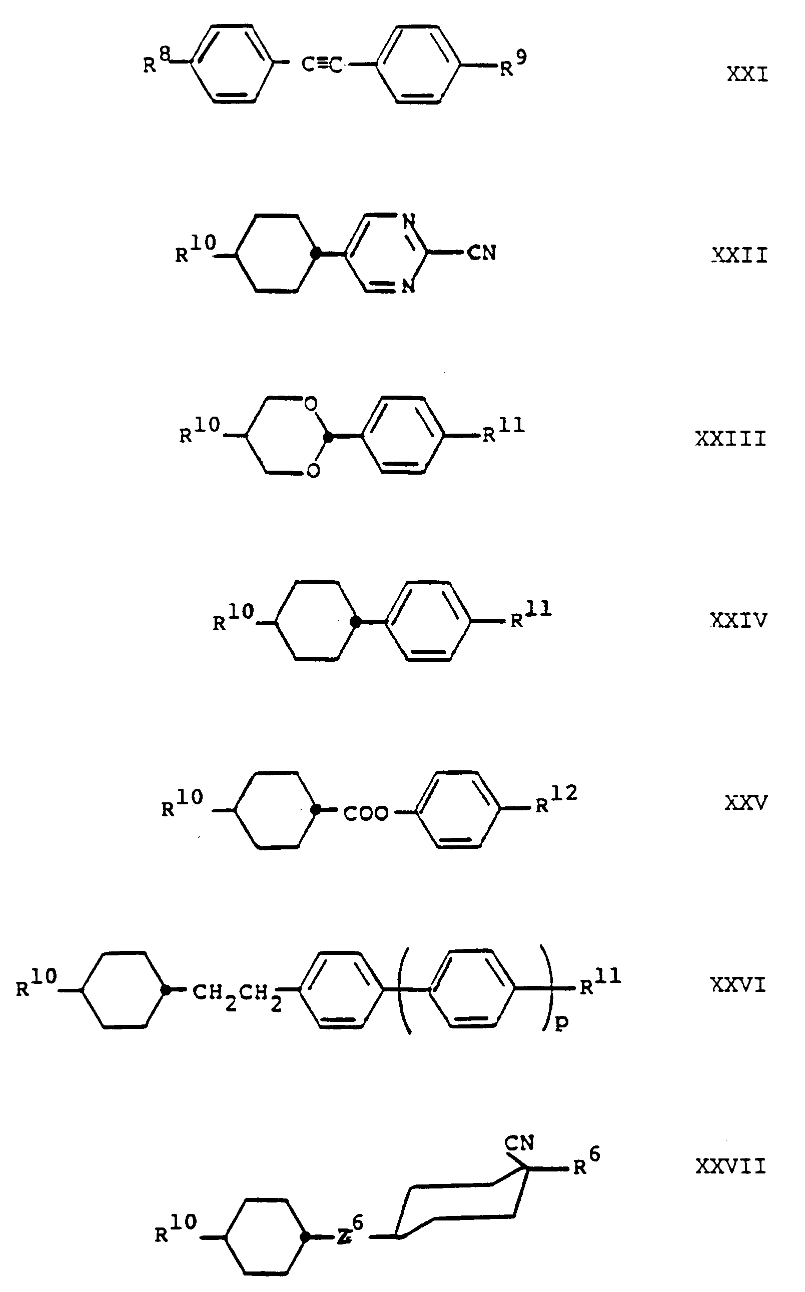 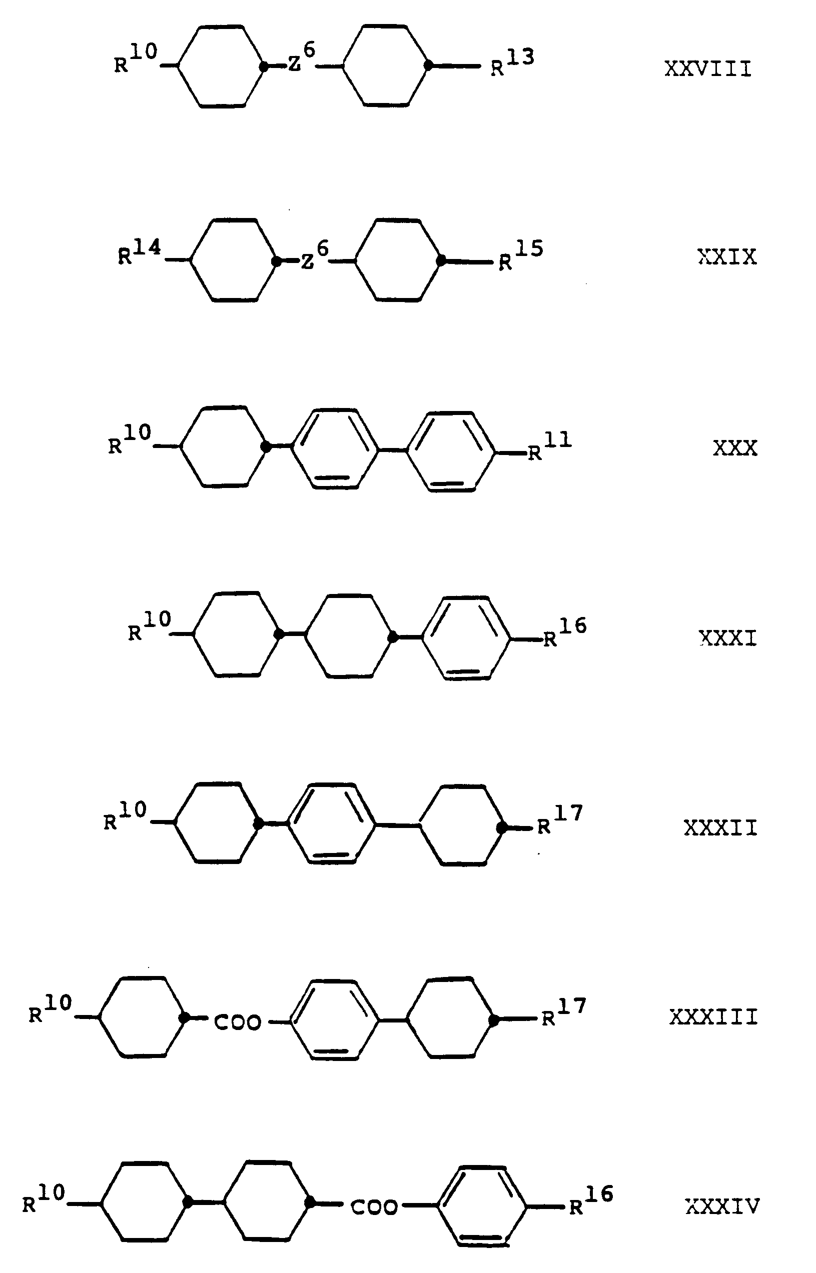 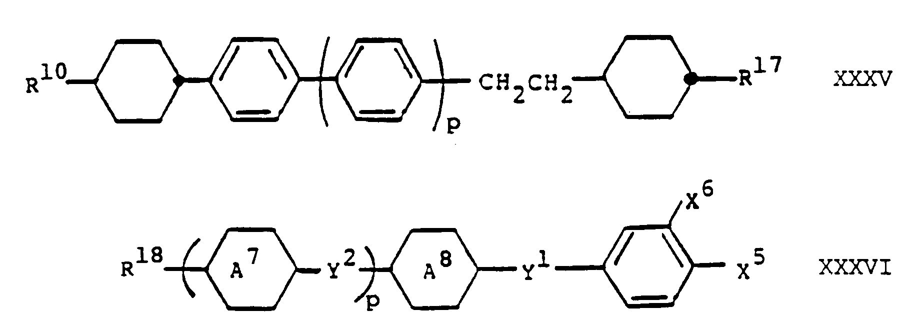
worin R⁶ Alkyl, 3E-Alkenyl oder 4-Alkenyl bedeutet; R⁷ Cyano oder Fluor darstellt; R⁸ und R⁹ Alkyl oder Alkoxy bezeichnen; R¹⁰ un R¹⁷ unabhängig voneinander Alkyl, 1E-Alkenyl, 3E-Alkenyl oder 4-Alkenyl bedeuten; R¹¹ Cyano, -NCS, Alkyl, 1E-Alkenyl, 3E-Alkenyl, 4-Alkenyl, Alkoxy, 2E-Alkenyloxy oder 3-Alkenyloxy bezeichnet; R¹² Alkoxy, 2E-Alkenyloxy oder 3-Alkenyloxy bedeutet; p für die Zahl O oder 1 steht; Z⁶ eine einfache Kovalenzbindung oder -CH₂CH₂- darstellt; R¹³ Cyano, Alkyl, 1E-Alkenyl, 3E-Alkenyl oder 4-Alkenyl bedeutet; R¹⁴ Alkyl, 1E-Alkenyl oder 4-Alkenyl bezeichnet; R¹⁵ Alkoxy, 2E-Alkenyloxy oder 3-Alkenyloxy darstellt; R¹⁶ Cyano, Alkyl, 1E-Alkenyl, 3E-Alkenyl, 4-Alkenyl, Alkoxy, 2E-Alkenyloxy oder 3-Alkenyloxy bezeichnet; X⁵ Fluor oder Chlor und X⁶ Wasserstoff, Fluor oder Chlor bezeichnen; R¹⁸ Alkyl, 3E-Alkenyl, 4-Alkenyl, Alkoxy, 2E-Alkenyloxy oder 3-Alkenyloxy bedeutet; eine der Gruppen Y¹ und Y² eine einfache Kovalenzbindung, -COO-, -OOC-, -CH₂CH₂-, -CH₂O- oder -OCH₂- und die andere der Gruppen Y¹ und Y² eine einfache Kovalenzbindung bedeutet; und die Ringe A⁷ und A⁸ unabhängig voneinander trans-1,4-Cyclohexylen, in welchem gegebenenfalls 2 nicht benachbarte CH₂-Gruppen durch Sauerstoff ersetzt sind, oder 1,4-Phenylen, in welchem gegebenenfalls 1 oder 2 CH₂ -Gruppen durch Stickstoff ersetzt sind, darstellen.

Vorzugsweise besitzen die Reste R⁶ und R⁸-R¹⁸ höchstens je 12 Kohlenstoffatome, insbesondere höchstens je 7 Kohlenstoffatome.

Die erfindungsgemässen Gemische für smektische Anwendungen (insbesondere für getiltete smektische oder chiral getiltete smektische Phasen) enthalten neben einer oder mehreren Verbindungen der Formel I-1 vorzugsweise eine oder mehrere Verbindungen aus der Gruppe der Verbindungen der allgemeinen Formeln 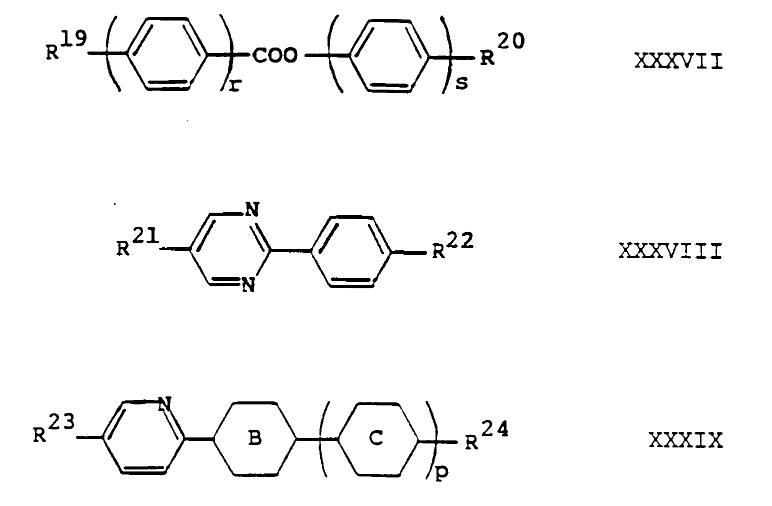 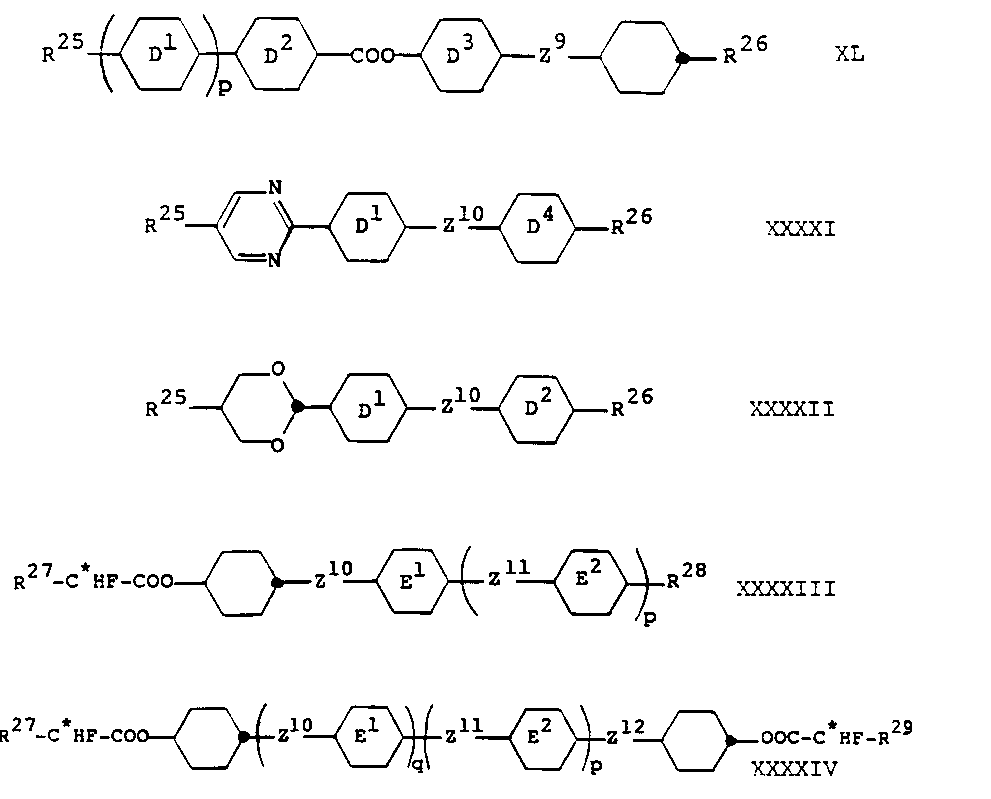
worin R¹⁹ und R²⁰ Alkyl, Alkoxy, Alkenyloxy, Alkanoyloxy oder Alkoxycarbonyl mit bis zu 18 Kohlenstoffatomen bezeichnen; r und s unabhängig voneinander 1 oder 2 bedeuten; R²¹ und R²² Alkyl, Alkoxy oder Alkenyloxy mit bis zu 18 Kohlenstoffatomen bezeichnen; Ring B unsubstituiertes oder mit Halogen und/oder Methyl substituiertes 1,4-Phenylen darstellt; Ring C trans-1,4-Cyclohexylen oder unsubstituiertes oder mit Halogen und/oder Methyl substituiertes 1,4-Phenylen darstellt; p und q unabhängig voneinander für die Zahl O oder 1 stehen; R²³ und R²⁴ unabhängig voneinander eine unsubstituierte oder mit Halogen substituierte C₁-C₁₈-Alkyl- oder C₂-C₁₈-Alkenylgruppe bezeichnen, in welcher gegebenenfalls eine oder zwei nicht benachbarte CH₂-Gruppen durch -O-, -COO- und/oder -OOC- ersetzt sind; die Ringe D¹, D² und D³ unabhängig voneinander unsubstituiertes oder mit Cyano, Halogen oder Niederalkyl substituiertes 1,4-Phenylen darstellen; Z⁹ eine einfache Kovalenzbindung, -CH₂CH₂-, -OCH₂-, -COO- oder -OOC-bezeichnet; R²⁵ und R²⁶ unabhängig voneinander eine unsubstituierte oder halogensubstituierte C₁-C₁₈-Alkyl- oder C₂-C₁₈-Alkenylgruppe bedeuten, in welcher gegebenenfalls eine oder zwei nicht benachbarte CH₂-Gruppen durch Sauerstoff ersetzt sind; Ring D⁴ trans-1,4-Cyclohexylen oder unsubstituiertes oder mit Cyano, Halogen oder Niederalkyl substituiertes 1,4-Phenylen darstellt; Z¹⁰, Z¹¹ und Z¹² unabhängig voneinander eine einfache Kovalenzbindung, -COO-, -OOC-, -CH₂CH₂-, -OCH₂- oder -CH₂O- bezeichnen; R²⁷ und R²⁹ unabhängig voneinander eine C₁-C₁₅-Alkyl-oder C₂-C₁₅-Alkenylgruppe bedeuten, in welcher gegebenenfalls eine CH₂-Gruppe durch Sauerstoff ersetzt ist; R²⁸ eine unsubstituierte oder mit Halogen substituierte C₁-C₁₅-Alkyl- oder C₂-C₁₅-Alkenylgruppe bezeichnet, in welcher gegebenenfalls eine CH₂-Gruppe durch Sauerstoff ersetzt ist und/oder gegebenenfalls eine CH₂-Gruppe durch eine Estergruppe -COO- oder -OOC- ersetzt ist; die Ringe E¹ und E² unabhängig voneinander unsubstituiertes oder mit Halogen, Cyano und/oder Methyl substituiertes 1,4-Phenylen darstellen, in welchem gegebenenfalls 1 oder 2 CH-Gruppen durch Stickstoff ersetzt sind; und C* ein chirales Kohlenstoffatom bezeichnet.

Die Herstellung der flüssigkristallinen Gemische und der elektro-optischen Vorrichtungen kann in an sich bekannter Weise erfolgen.

Die Erfindung wird durch die folgenden Beispiele weiter veranschaulicht. Die optischen Antipoden von chiralen Verbindungen besitzen jeweils die gleichen Phasenumwandlungstemperaturen und absolut gleiche Werte der Verdrillung aber mit entgegengesetzten Vorzeichen. Die zur Charakterisierung der Phasenumwandlungen verwendeten Abkürzungen stehen für folgende Bedeutungen:
- C: für kristallin
- S: für smektisch
- S_{A}, S_{B}, S_{C}: etc. für smektisch A, B, C etc.
- S_{C}*, S_{F}*: etc. für chiral smektisch C, F etc.
- N: für nematisch
- N*: für cholesterisch
- I: für isotrop.

### Beispiel 1

Ein Gemisch von 19 g 1-Butyloxy-4-[4-(trans-4-pentylcyclohexyl)-1-butenyl]benzol (cis/trans-Gemisch), 200 ml absolutem Toluol und 100 ml absolutem Aethylacetat wurde mit 2 g Palladium/Kohle (10%) versetzt und bei Normaldruck und Raumtemperatur bis zum Stillstand der Wasserstoffaufnahme hydriert. Das anorganische Material wurde abfiltriert und das Filtrat eingeengt. Der Rückstand wurde an Kieselgel mit Toluol/Aethylacetat chromatographisch gereinigt. Umkristallisation aus Aethanol ergab 16 g reines 1-Butyloxy-4-[4-(trans-4-pentylcyclohexyl)-1-butyl]benzol mit Smp. (C-S_{B}) 29°C und Klp. (S_{B}-I) 43°C.

Das als Ausgangsmaterial verwendete 1-Butyloxy-4-[4-(trans-4-pentyloxyhexyl)-1-butenyl]benzol wurde wie folgt hergestellt:
Ein Gemisch von 54 g (4-Butyloxyphenyl)methyltriphenylphosphoniumbromid und 450 ml absolutem t-Butylmethyläther wurde bei 0°C und unter Stickstoffbegasung mit 12 g festem Kalium-t-butylat versetzt. Nach beendeter Zugabe wurde das Reaktionsgemisch noch 15 Minuten bei 0°C gerührt (wobei eine tieforange Färbung entstand) und dann innert 30 Minuten bei 0°C mit einer Lösung von 15 g 3-(trans-4-Pentylcyclohexyl)propionaldehyd auf 150 ml absolutem t-Butylmethyläther versetzt. Anschliessend wurde das Reaktionsgemisch über Nacht bei Raumtemperatur gerührt und dann auf 1000 ml Wasser gegossen und dreimal mit je 200 ml Diäthyläther extrahiert. Die organischen Phasen wurden noch zweimal mit je 500 ml konzentrierter Kochsalz-Lösung gewaschen, über Magnesiumsulfat getrocknet, filtriert und anschliessend eingeengt. Chromatographie des Rückstandes an Kieselgel mit Hexan/ Toluol (Vol. 4:1) ergab 21 g 1-Butyloxy-4-[4-(trans-4-Pentylcyclohexyl)-1-butenyl]benzol (cis/trans-Gemisch). Umkristallisation aus Aethanol ergab reines trans-Isomer mit Smp. (C-S_{B}) 46°C, S_{B}-S_{A} 74°C, S_{A}-N 76°C und Klp. (N-I) 91°C.

In analoger Weise können folgende Verbindungen hergestellt werden:
1-Fluor-4-[4-(trans-4-pentylcyclohexyl)-1-butyl]benzol, Smp. (C-I) 13°C, Klp. (N-I) -17°C;
1-Chlor-4-[4-(trans-4-pentylcyclohexyl)-1-butyl]benzol;
1-Brom-4-[4-(trans-4-pentylcyclohexyl)-1-butyl]benzol;
1-Jod-4-[4-(trans-4-pentylcyclohexyl)-1-butyl]benzol;
1,2-Difluor-4-[4-(trans-4-pentylcyclohexyl)-1-butyl]benzol, Smp. (C-I) 6°C;
1-Methyl-4-[4-(trans-4-pentylcyclohexyl)-1-butyl]benzol;
1-(Trifluormethyl)-4-[4-(trans-4-pentylcyclohexyl)-1-butyl]benzol;
1-(Trifluormethoxy)-4-[4-(trans-4-pentylcyclohexyl)-1-butyl]benzol, Smp. (C-I) 18°C, Klp. (N-I) -12°C;
4-[4-(trans-4-Pentylcyclohexyl)-1-butyl]benzonitril, Smp. (C-I) 51°C, Klp. (N-I) 39°C;
1-Fluor-4-[4-(trans-4-[trans-4-propylcyclohexyl]cyclohexyl)-1-butyl]benzol, Smp. (C-S) 56°C, S-N 87°C, Klp. (N-I) 116°;
1,2-Difluor-4-[4-(trans-4-[trans-4-propylcyclohexyl]cyclohexyl)-1-butyl]benzol, Smp. (C-S) 39°C, S-N 69°C, Klp. (N-I) 103°C;
1-Methyl-4-[4-(trans-4-[trans-4-propylcyclohexyl]cyclohexyl)-1-butyl]benzol;
1-(Trifluormethyl)-4-[4-(trans-4-[trans-4-propylcyclohexyl]cyclohexyl)-1-butyl]benzol;
1-(Trifluormethoxy)-4-[4-(trans-4-[trans-4-propylcyclohexyl]cyclohexyl)-1-butyl]benzol, Smp. (C-S) 47°C, S-N 86°C, Klp. (N-I) 111°C;
4-[4-(trans-4-[trans-4-Propylcyclohexyl]cyclohexyl)-1-butyl]benzonitril;
2-Fluor-4-[4-(trans-4-[trans-4-propylcyclohexyl]cyclohexyl)-1-butyl]benzonitril;
1-Aethoxy-2,3-difluor-4-[4-(trans-4-[trans-4-propylcyclohexyl]cyclohexyl)-1-butyl]benzol;
1-Fluor-4-[4-(trans-4-[trans-4-pentylcyclohexyl]cyclohexyl)-1-butyl]benzol;
1,2-Difluor-4-[4-(trans-4-[trans-4-pentylcyclohexyl]cyclohexyl)-1-butyl]benzol;
1-Methyl-4-[4-(trans-4-[trans-4-pentylcyclohexyl]cyclohexyl)-1-butyl]benzol;
1-(Trifluormethyl)-4-[4-(trans-4-[trans-4-pentylcyclohexyl]cyclohexyl)-1-butyl]benzol;
1-(Trifluormethoxy)-4-[4-(trans-4-[trans-4-pentylcyclohexyl]cyclohexyl)-1-butyl]benzol;
4-[4-(trans-4-[trans-4-Pentylcyclohexyl]cyclohexyl)-1-butyl]benzonitril;
2-Fluor-4-[4-(trans-4-[trans-4-pentylcyclohexyl]cyclohexyl)-1-butyl]benzonitril;
1-Aethoxy-2,3-difluor-4-[4-(trans-4-[trans-4-pentylcyclohexyl]cyclohexyl)-1-butyl]benzol.

### Beispiel 2

Ein Gemisch von 0,3 g 4-[4-(trans-4-Pentylcyclohexyl)-1-butyl]phenol, 0,14 g Methyljodid, 0,6 g Kaliumcarbonat und 50 ml absolutem Butanon wurde über Nacht zum Rückfluss erhitzt. Anschliessend wurde das abgekühlte Reaktionsgemisch auf Wasser gegossen und dreimal mit je 50 ml Diäthyläther extrahiert. Die vereinigten organischen Phasen wurde mit 500 ml Wasser gewaschen, über Magnesiumsulfat getrocknet, filtriert und eingeengt. Chromatographie des Rückstandes an Kieselgel mit Toluol und Umkristallisation aus Aethanol ergab reines 1-Methoxy-4-[4-(trans4-pentylcyclohexyl)-1-butyl]benzol mit Smp. (C-I) 27°C und Klp. (S_{B}-I) 16°C.

Das als Ausgangsmaterial verwendete 4-[4-(trans-4-Pentylcyclohexyl)-1-butyl]phenol wurde wie folgt hergestellt:
Ein Gemisch von 16 g 1-Butyloxy-4-[4-(trans-4-pentylcyclohexyl)-1-butyl]benzol und 100 ml absolutem Dichlormethan wurde bei 0°C und unter Stickstoffbegasung mit 50 ml einer 1M Lösung von Bortribromid in Dichlormethan versetzt. Nach beendeter Zugabe wurde das Reaktionsgemisch noch 2 Stunden bei Raumtemperatur gerührt und dann vorsichtig mit 200 ml Wasser versetzt. Die organische Phase wurde abgetrennt und die wässrige Phase dreimal mit je 100 ml Dichlormethan nachextrahiert. Die vereinigten organischen Phasen wurden nacheinander mit 1000 ml Wasser, 500 ml verdünnter Kaliumcarbonat-Lösung und nochmals mit 1000 ml Wasser gewaschen, über Magnesiumsulfat getrocknet, filtriert und anschliessend eingeengt. Umkristallisation des Rückstandes aus Hexan ergab 11 g reines 4-[4-(trans-4-Pentylcyclohexyl)-1-butyl]phenol mit Smp. 86°C.

In analoger Weise können folgende Verbindungen hergestellt werden:
1-Aethoxy-4-[4-(trans-4-pentylcyclohexyl)-1-butyl]benzol, Smp. (C-S_{B}) 18°C, Klp. (S_{B}-I) 31°C;
1-Propyloxy-4-[4-(trans-4-pentylcyclohexyl)-1-butyl]benzol, Smp. (C-I) 39°C, Klp. (S_{B}-I) 28°C;
1-Pentyloxy-4-[4-(trans-4-pentylcyclohexyl)-1-butyl]benzol, Smp. (C-S_{B}) 31°C, Klp. (S_{B}-I) 39°C;
1-Hexyloxy-4-[4-(trans-4-pentylcyclohexyl)-1-butyl]benzol;
1-Heptyloxy-4-[4-(trans-4-pentylcyclohexyl)-1-butyl]benzol;
1-Octyloxy-4-[4-(trans-4-pentylcyclohexyl)-1-butyl]benzol;
1-[(trans-4-Pentylcyclohexyl)methoxy]-4-[4-(trans-4-pentylcyclohexyl)-1-butyl]benzol, Smp. (C-S_{B}) 42°C, Klp. (S_{B}-I) 108°C;
1-[3-(trans-4-Pentylcyclohexyl)-1-propyloxy]-4-[4-(trans-4-pentylcyclohexyl)-1-butyl]benzol, Smp. (C-S_{B}) 52°C, Klp. (S_{B}-I) 94°C;
1-[3E-(trans-4-Pentylcyclohexyl)allyloxy]-4-[4-(trans-4-pentylcyclohexyl)-1-butyl]benzol, Smp. (C-S) 77°C, S-S_{B} 94°C, S_{B}-N 99°C, Klp. (N-I) 107°C.

### Beispiel 3

1,9 g 2,3-Difluor-4-heptyloxybenzoesäure, 2 g 4-[4-(trans-4-Pentylcyclohexyl)-1-butyl]phenol und 0,1 g 4-(Dimethylamino)pyridin wurden in 250 ml Dichlormethan gelöst und die Lösung innert 10 Minuten unter Rühren portionenweise mit 1,6 g N,N′-Dicyclohexylcarbodiimid versetzt. Das Gemisch wurde über Nacht bei Raumtemperatur gerührt und dann filtriert. Das Filtrat wurde mit Dichlormethan verdünnt, zweimal mit je 50 ml gesättigter Natriumcarbonat-Lösung und dann mit Wasser gewaschen, über Magnesiumsulfat getrocknet, filtriert und dann eingeengt. Chromatographie des Rückstandes an Kieselgel mit Toluol/Aethylacetat (Vol. 4:1) und Umkristallisation aus Aethanol ergab 2 g 2,3-Difluor-4-heptyloxybenzoesäure-4-[4-(trans-4-pentylcyclohexyl)-1-butyl]phenylester mit Smp. (C-S_{C}) 67°C, S_{C}-N 70°C und Klp. (N-I) 128°C.

In analoger Weise können folgende Verbindungen hergestellt werden:
2,3-Difluor-4-octyloxybenzoesäure-4-[4-(trans-4-pentylcyclohexyl)-1-butyl]phenylester, Smp. (C-S_{C}) 70°C, S_{C}-N 82°C, Klp. (N-I) 123°C;
2,3-Difluor-4-nonyloxybenzoesäure-4-[4-(trans-4-pentylcyclohexyl)-1-butyl]phenylester, Smp. (C-S_{C}) 71°C, S_{C}-N 91°C, Klp. (N-I) 121°C;
2,3-Difluor-4-decyloxybenzoesäure-4-[4-(trans-4-pentylcyclohexyl)-1-butyl]phenylester, Smp. (C-S_{C}) 69°C, S_{C}-N 96°C, Klp. (N-I) 120°C;
2,3-Difluor-4-undecyloxybenzoesäure-4-[4-(trans-4-pentylcyclohexyl)-1-butyl]phenylester, Smp. (C-S_{C}) 58°C, S_{C}-N 103°C, Klp. (N-I) 119°C;
2,3-Difluor-4-dodecyloxybenzoesäure-4-[4-(trans-4-pentylcyclohexyl)-1-butyl]phenylester, Smp. (C-S_{C}) 56°C, S_{C}-N 107°C, Klp. (N-I) 118°C;
trans-4-Pentylcyclohexancarbonsäure-4-[4-(trans-4-pentylcyclohexyl)-1-butyl]phenylester, Smp. (C-S_{B}) 59°C, S_{B}-N 135°C, Klp. (N-I) 139°C;
4-[4-(trans-4-Pentylcyclohexyl)-1-butyl]-4′-[2(R)-fluorhexanoyl oxy]biphenyl, Smp. (C-S_{B}) 68°C, Klp. (S_{B}-I) 140°C.

### Beispiel 4

2 g 4-[4-(trans-4-Pentylcyclohexyl)-1-butyl]benzoesäure, 0,7 g 4-Fluorphenol, 1,5 g N,N′-Dicyclohexylcarbodiimid, 0,1 g 4-(Dimethylamino)pyridin und 100 ml Dichlormethan wurden in analoger Weise zu Beispiel 3 umgesetzt. Dies ergab 1,8 g 4-[4-(trans-4-Pentylcyclohexyl)-1-butyl]benzoesäure-4-fluorphenylester mit Smp. (C-N) 63°C und Klp. (N-I) 104°C.

Die als Ausgangsmaterial verwendete 4-[4-(trans-4-Pentylcyclohexyl)-1-butyl]benzoesäure wurde wie folgt hergestellt:
Ein Gemisch von 36 g 4-[4-(trans-4-Pentylcyclohexyl)-1-butyl]benzonitril, 300 ml konzentrierter Schwefelsäure, 300 ml Eisessig und 300 ml Wasser wurde über Nacht auf 120°C erhitzt. Das abgekühlte Reaktionsgemisch wurde auf 2000 ml Wasser gegossen und der entstandene Niederschlag abgenutscht, trocken gesaugt, mit viel Wasser gewaschen und anschliessend aus Aethanol umkristallisiert. Dies ergab 28 g reine 4-[4-(trans-4-Pentylcyclohexyl)-1-butyl]benzoesäure mit Smp. (C-N) 195°C und Klp. (N-I) 199°C.

In analoger Weise können folgende Verbindungen hergestellt werden:
4-[4-(trans-4-Pentylcyclohexyl)-1-butyl]benzoesäure-4-chlorphenylester, Smp. (C-N) 75°C, Klp. (N-I) 126°C;
4-[4-(trans-4-Pentylcyclohexyl)-1-butyl]benzoesäure-4-bromphenylester, Smp. (C-N) 80°C, S_{A}-N 58°C, Klp. (N-I) 128°C;
4-[4-(trans-4-Pentylcyclohexyl)-1-butyl]benzoesäure-4-jodphenylester, Smp. (C-N) 92°C, Klp. (N-I) 125°C;
4-[4-(trans-4-Pentylcyclohexyl)-1-butyl]benzoesäure-4-cyanophenylester, Smp. (C-N) 78°C, Klp. (N-I) 151°C;
4-[4-(trans-4-Pentylcyclohexyl)-1-butyl]benzoesäure-2-fluor-4-cyanophenylester;
4-[4-(trans-4-Pentylcyclohexyl)-1-butyl]benzoesäure-3-fluor-4-cyanophenylester, Smp. (C-N) 59°C, Klp. (N-I) 132°C;
4-[4-(trans-4-Pentylcyclohexyl)-1-butyl]benzoesäure-3,4-difluorphenylester, Smp. (C-N) 50°C, Klp. (N-I) 88°C;
4-[4-(trans-4-Pentylcyclohexyl)-1-butyl)benzoesäure-2,3-difluor-4-äthoxyphenylester;
4-[4-(trans-4-Pentylcyclohexyl)-1-butyl]benzoesäure-4-methylphenylester;
4-[4-(trans-4-Pentylcyclohexyl)-1-butyl]benzoesäure-4-propylphenylester;
4-[4-(trans-4-Pentylcyclohexyl)-1-butyl]benzoesäure-4-pentylphenylester;
4-[4-(trans-4-Pentylcyclohexyl)-1-butyl]benzoesäure-trans-4-propylcyclohexylester;
4-[4-(trans-4-Pentylcyclohexyl)-1-butyl]benzoesäure-4-trifluormethoxyphenylester, Smp. (C-N) 71°C, Klp. (N-I) 109°C.

### Beispiel 5

Eine Lösung von 3 g 4-[4-(trans-4-Pentylcyclohexyl)-1-butyl]benzaldehyd und 1,7 g 2-Pentyl-1,3-propandiol in 50 ml Toluol wurde mit 2 Tropen 10%iger (Vol.) Schwefelsäure versetzt. Das Gemisch wurde 2 Stunden zum Sieden erhitzt, wobei das entstandene Wasser gleichzeitig abdestilliert wurde. Dann wurden 4 Tropen Triäthylamin zum Reaktionsgemisch zugegeben. Nach dem Erkalten wurde das Gemisch mit 50 ml 1N Natriumhydrogencarbonat-Lösung und zweimal mit je 50 ml Wasser gewaschen, über Magnesiumsulfat getrocknet, filtriert und anschliessend eingeengt. Chromatographie des Rückstandes an Kieselgel mit Toluol und Umkristallisation aus Aceton ergab 1,2 g trans-5-Pentyl-2-(4-[4-(trans-4-pentylcyclohexyl)-1-butyl]phenyl-1,3-dioxan mit Smp. (C-N) 73°C und Klp. (N-I) 106°C.

Der als Ausgangsmaterial verwendete 4-[4-(trans-4-Pentylcyclohexyl)-1-butyl]benzaldehyd wurde wie folgt hergestellt:
Eine Lösung von 10 g 4-[4-(trans-4-Pentylcyclohexyl)-1-butyl]benzonitril in 200 ml absolutem Dichlormethan wurde bei -78°C und unter Stickstoffbegasung mit 50 ml einer Lösung von Diisobutylaluminiumhydrid in Hexan (Vol. 20%) versetzt. Nach beendeter Zugabe wurde das Reaktionsgemisch noch 8 Stunden bei Raumtemperatur gerührt und dann mit 200 ml Wasser versetzt und dreimal mit je 100 ml Dichlormethan extrahiert. Die vereinigten organischen Phasen wurden zweimal mit je 500 ml Wasser gewaschen, über Magnesiumsulfat getrocknet, filtriert und eigeengt. Dies ergab 7,5 g 4-[4-(trans-4-Pentylcyclohexyl)-1-butyl]benzaldehyd.

In analoger Weise können folgende Verbindungen hergestellt werden:
trans-5-Propyl-2-(4-[4-(trans-4-propylcyclohexyl)-1-butyl]phenyl)-1,3-dioxan;
trans-5-Propyl-2-(4-[4-(trans-4-pentylcyclohexyl)-1-butyl]phenyl)-1,3-dioxan;
trans-5-Pentyl-2-(4-[4-(trans-4-propylcyclohexyl)-1-butyl]phenyl)-1,3-dioxan.

### Beispiel 6

Ein Gemisch von 1,3 g Lithiumaluminiumhydrid und 100 ml absolutem Diäthyläther wurde bei 0°C und unter Stickstoffbegasung vorgelegt und mit einer Lösung von 10 g Aluminiumchlorid und 200 ml absolutem Diäthyläther versetzt. Nach beendeter Zugabe wurde das Gemisch noch 20 Minuten bei Raumtemperatur gerührt und innert 30 Minuten mit einer Lösung von 9 g 1-(trans-4-Pentylcyclohexyl)-4-[4-(trans-4-pentylcyclohexyl)butanoyl]benzol und 100 ml absolutem Dichlormethan versetzt. Nach beendeter Zugabe wurde das Reaktionsgemisch über Nacht unter leichtem Rückfluss erhitzt, dann auf 0°C abgekühlt und mit 50 ml Wasser und 500 ml 25%iger Salzsäure versetzt. Die organische Phase wurde abgetrennt und die wässrige Phase dreimal mit je 100 ml Diäthyläther nachextrahiert. Die vereinigten organischen Phasen wurden mit 500 ml Wasser, 250 ml konzentrierter Kaliumcarbonat-Lösung und nochmals mit 500 ml Wasser gewaschen, über Magnesiumsulfat getrocknet, filtriert und anschliessend eingeengt. Chromatographie des Rückstandes an Kieselgel mit Toluol und Umkristallisation aus Aceton ergab 5,7 g 1-(trans-4-Pentylcyclohexyl)-4-[4-(trans-4-pentylcyclohexyl)-1-butyl]benzol mit Smp. (C-S_{B}) 50°C und Klp. (S_{B}-I) 111°C.

Das als Ausgangsmaterial verwendete 1-(trans-4-Pentylcyclohexyl)-4-[4-(trans-4-pentylcyclohexyl)-butanoyl]benzol wurde wie folgt hergestellt:
0,6 g Magnesiumspäne wurden vorgelegt und unter Stickstoffbegasung mit 5 ml absolutem Diäthyläther überschichtet und dann nach Zugabe eines Jodkristalls mit einer Lösung von 7 g 3-(trans-4-Pentylcyclohexyl)-1-propylbromid in 50 ml absolutem Diäthyläther versetzt. Nach beendeter Zugabe wurde das Gemisch noch während 30 Minuten zum Rückfluss erhitzt. Das Reaktionsgemisch wurde dann mit einer Lösung von 2,6 g 4-(trans-4-Pentylcyclohexyl)benzonitril in 50 ml absolutem Diäthyläther versetzt und anschliessend über Nacht unter leichtem Rückfluss erhitzt. Das abgekühlte Reaktionsgemisch wurde mit 200 ml 25%iger Salzsäure versetzt und die organische Phase abgetrennt. Die wässrige Phase wurde dreimal mit je 50 ml Diäthyläther extrahiert. Die vereinigten organischen Phasen wurden mit 500 ml Wasser, 250 ml konzentrierter Kaliumcarbonat-Lösung und nochmals mit 500 ml Wasser gewaschen, über Magnesiumsulfat getrocknet, filtriert und dann eingeengt. Umkristallisation des Rückstandes aus Aethanol ergab 4,1 g 1-(trans-4-Pentylcyclohexyl)-4-[4-(trans-4-pentylcyclohexyl)-butanoyl]benzol mit Smp. (C-S_{B}) 75°C, S_{B}-N 89°C und Klp. (N-I) 116°C.

In analoger Weise können folgende Verbindungen hergestellt werden:
1-(trans-4-Propylcyclohexyl)-4-[4-(trans-4-propylcyclohexyl)-1-butyl]benzol;
1-(trans-4-Propylcyclohexyl)-4-[4-(trans-4-pentylcyclohexyl)-1-butyl]benzol;
1-(trans-4-Pentylcyclohexyl)-4-[4-(trans-4-propylcyclohexyl)-1-butyl]benzol;
4-(trans-4-Propylcyclohexyl)-4′-[4-(trans-4-propylcyclohexyl)-1-butyl]biphenyl;
4-(trans-4-Propylcyclohexyl)-4′-[4-(trans-4-pentylcyclohexyl)-1-butyl]biphenyl;
4-[2(S)-Methylbutyloxy]-4′-[4-(trans-4-pentylcyclohexyl)-1-butyl]biphenyl;
1-[2-(trans-4-Pentylcyclohexyl)-1-äthyl]-4-[4-(trans-4-pentylcyclohexyl)-1-butyl]benzol;
2-(4-[4-(trans-4-Pentylcyclohexyl)-1-butyl]phenyl-5-propylpyridin;
2-(4-[4-(trans-4-Pentylcyclohexyl)-1-butyl]phenyl-5-butylpyridin;
2-(4-[4-(trans-4-Pentylcyclohexyl)-1-butyl]phenyl-5-pentylpyridin;
2-(4-[4-(trans-4-Pentylcyclohexyl)-1-butyl]phenyl-5-hexylpyridin;
2-(4-[4-(trans-4-Pentylcyclohexyl)-1-butyl]phenyl-5-heptylpyridin;
2-(4-[4-(trans-4-Pentylcyclohexyl)-1-butyl]phenyl-5-octylpyridin;
2-(4-[4-(trans-4-Pentylcyclohexyl)-1-butyl]phenyl-5-nonylpyridin;
2-(4-[4-(trans-4-Pentylcyclohexyl)-1-butyl]phenyl-5-decylpyridin;
1-(4-[4-(trans-4-Pentylcyclohexyl)-1-butyl]phenyl-4-pentylbicyclo[2.2.2]octan;
(4aαH,8aβH)-Decahydro-2α-(4-[4-(trans-4-Pentylcyclohexyl)-1-butyl]phenyl)-6β-pentylnaphthalin.

### Beispiel 7

Eine Lösung von 4 g 4-Hydroxy-4′-[4-(trans-4-Pentylcyclohexyl)-1-butyl]biphenyl, 1,3 g L(-)-Milchsäureäthylester, 2,9 g Triphenylphosphin und 100 ml Tetrahydrofuran wurde mit 1,9 g Azodicarbonsäurediäthylester versetzt. Das Reaktionsgemisch wurde über Nacht bei Raumtemperatur gerührt, dann eingeengt und mit 100 ml heissem Hexan aufgeschlämmt. Der dabei entstandene Niederschlag wurde abfiltriert und das Filtrat eingeengt. Chromatographie des Rückstandes an Kieselgel mit Toluol/Aethylacetat (Vo. 4:1) und Umkristallisation aus Aethanol ergab 2,9 g reines Aethyl-2(R)-[4′-[4-(trans-4-pentylcyclohexyl)-1-butyl]-4-biphenylyloxy]propionat mit Smp. (C-S_{B}) 71°C und Klp. (S_{B}-I) 76°C.

Das als Ausgangsmaterial verwendete 4-Hydroxy-4′-[4-(trans-4-Pentylcyclohexyl)-1-butyl]biphenyl wurde wie folgt hergestellt:
Eine Lösung von 2,5 g (S)-4-(2-Methylbutyl)oxy-4′-[4-(trans-4-pentylcyclohexyl)-1-butyl]biphenyl und 100 ml absolutem Dichlormethan wurde in analoger Weise zu Beispiel 2 mit 7 ml einer 1M Lösung von Bortribromid und Dichlormethan umgesetzt. Dies ergab 2,2 g reines 4-Hydroxy-4′-[4-(trans-4-Pentylcyclohexyl)-1-butyl]-biphenyl mit Smp. 175°C.

In analoger Weise können folgende Verbindungen hergestellt werden:
Butyl-2(R)-[4′-[4-(trans-4-pentylcyclohexyl)-1-butyl]-4-biphenylyloxy]propionat, Smp. (C-S_{B}) 59°C, Klp. (S_{B}-I) 69°C;
Aethyl-2(R)-[4′-[4-(trans-4-propylcyclohexyl)-1-butyl]-4-biphenylyloxy]propionat;
Butyl-2(R)-[4′-[4-(trans-4-pentylcyclohexyl)-1-butyl]-4-biphenylyloxy]propionat.

### Beispiel 8

Unter Stickstoffatmosphäre wurde ein Gemisch aus 0,46 g 2-Decylmalonaldehyd-tetramethylacetal, 0,02 g p-Toluolsulfonsäure-monohydrat und 0,5 ml Wasser 30 Minuten auf 70-80°C erhitzt und dann mit 0,5 g Natriumhydrogencarbonat versetzt. Das Gemisch wurde noch 5 Minuten gerührt und dann filtriert. Der Filterrückstand wurde mit Methanol nachgewaschen. Das Filtrat enthaltend rohes 2-(Methoxymethyliden)dodecanal wurde unter Stickstoff zu einem Gemisch aus 0,4 g 4-[4-(trans-4-Pentylcyclohexyl)-1-butyl]benzamidin-hydrochlorid, 10 ml Methanol und einer Natriummethylat-Lösung (hergestellt aus 0,42 ml Methanol und 0,12 g Natrium) zugetropft. Das Reaktionsgemisch wurde über Nacht gerührt und anschliessend mit konzentrierter Salzsäure auf pH 3-4 gestellt und dreimal mit je 50 ml Dichlormethan extrahiert. Die vereinigten organischen Phasen wurden zweimal mit je 100 ml Wasser gewaschen, über Magnesiumsulfat getrocknet, filtriert und anschliessend eingeengt. Der feste Rückstand wurde über Kieselgel mit Toluol chromatographisch gereinigt. Umkristallisation aus Aethanol ergab 0,4 g reines 5-Decyl-2-[4-[4-(trans-4-Pentylcyclohexyl)-1-butyl]phenyl]pyrimidin mit Smp. (C-S) 66°C, S-S_{C} 71°C, S_{C}-S_{A} 86°C, S_{A}-N 102°C, Klp. (N-I) 109°C.

Das als Ausgangsmaterial verwendete 4-[4-(trans-4-Pentylcyclohexyl)-1-butyl]benzamidin-hydrochlorid wurde wie folgt hergestellt:
a) Eine Lösung von 11,6 g 4-[4-(trans-4-Pentylcyclohexyl)-1-butyl]benzonitril in 7,6 ml Aethanol und 100 ml Toluol wurde bei 0-5°C mit Chlorwasserstoff gesättigt und dann während 2 Tagen bei Raumtemperatur weiter gerührt. Danach wurde das Reaktionsgemisch eingeengt, in 300 ml Diäthyläther aufgeschlämmt und dann filtriert. Der Filterrückstand wurde mit Diäthyläther nachgewaschen und dann getrocknet. Dies ergab 6 g 4-[4-(trans-4-Pentylcyclohexyl)-1-butyl]phenyl-imidoäthyläther-hydrochlorid.
b) Eine Lösung von 6 g 4-[4-(trans-4-Pentylcyclohexyl)-1-butyl]phenyl-imidoäthyläther und 50 ml Aethanol wurde mit 11,2 ml einer gesättigten äthanolischen Ammoniak-Lösung versetzt. Das Reaktionsgemisch wurde noch 2 Tage bei Raumtemperatur gerührt und dann eingeengt. Der feste Rückstand wurde in 50 ml Aethanol gelöst und dann mit 600 ml Diäthyläther versetzt, wobei das Produkt als ein weisser, feiner Niederschlag ausfiel. Dieser Niederschlag wurde abfiltriert und getrocknet. Dies ergab 5,2 g 4-[4-(trans-4-Pentylcyclohexyl)-1-butyl]benzamidin-hydrochlorid.

In analoger Weise können folgende Verbindungen hergestellt werden:
5-Pentyl-2-[4-[4-(trans-4-Pentylcyclohexyl)-1-butyl]phenyl]pyrimidin, Smp. (C-N) 78°C, Klp. (N-I) 119°C;
5-Hexyl-2-[4-[4-(trans-4-Pentylcyclohexyl)-1-butyl]phenyl]pyrimidin, Smp. (C-N) 79°C, Klp. (N-I) 113°C;
5-Heptyl-2-[4-[4-(trans-4-Pentylcyclohexyl)-1-butyl]phenyl]pyrimidin, Smp. (C-N) 77°C, Klp. (N-I) 116°C;
5-Octyl-2-[4-[4-(trans-4-Pentylcyclohexyl)-1-butyl]phenyl]pyrimidin, Smp. (C-N) 69°C, S_{C}-N 64°C, Klp. (N-I) 110°C;
5-Nonyl-2-[4-[4-(trans-4-Pentylcyclohexyl)-1-butyl]phenyl]pyrimidin, Smp. (C-S_{C}) 74°C, S-S_{C} 62°C, S_{C}-S_{A} 82°C, S_{A}-N 97°C, Klp. (N-I) 113°C;
5-Undecyl-2-[4-[4-(trans-4-Pentylcyclohexyl)-1-butyl]phenyl]pyrimidin, Smp. (C-S_{C}) 48°C, S-S_{C} 71°C, S_{C}-S_{A} 81°C, S_{A}-N 106°C, Klp. (N-I) 110°C;
5-Dodecyl-2-[4-[4-(trans-4-Pentylcyclohexyl)-1-butyl]phenyl]pyrimidin.

## Patentansprüche

1. Verbindungen der allgemeinen Formel 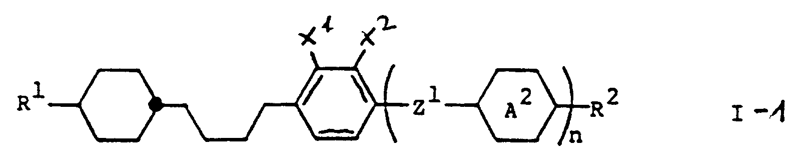 worin n für die Zahl O oder 1 steht; R¹ eine Gruppe R³ oder R³-A³-Z²- und R² eine Gruppe R⁴ oder R⁴-A⁴-Z³- bezeichnen; X¹ und X² unabhängig voneinander Wasserstoff oder Fluor bedeuten; A³ und A⁴ trans-1,4-Cyclohexylen bedeuten, und Ring A² unsubstituiertes oder mit Fluor mono- oder disubstituiertes 1,4-Phenylen oder trans-1,4-Cyclohexylen, trans-1,3-Dioxan-2,5-diyl, 1-Cyano-trans-1,4-cyclohexylen, Bicyclo[2.2.2]octan-1,4-diyl, Naphthalin-2,6-diyl, Tetralin-2,6-diyl oder trans-Decalin-2,6-diyl Pyridin-2,5-diyl, Pyrimidin-2,5-diyl oder Pyrazin-2,5-diyl darstellt; Z¹, Z² und Z³ je eine einfache Kovalenzbindung bedeuten, oder eine der Gruppen Z¹, Z² und Z³ auch -COO-, -OOC-, -CH₂O-, OCH₂-, -CH₂CH₂-, -C≡C-, -(CH₂)₃O-, -O(CH₂)₃-, -(CH₂)₄- oder die trans-Form von -CH=CH-CH₂O- oder -OCH₂-CH=CH- bedeuten; R³ und R⁴ unabhängig voneinander Halogen, Cyano, -NCS, -CF₃, -OCF₃ oder Alkyl bezeichnen, in welchem gegebenenfalls eine >CH-CH< durch >C=C< ersetzt ist und/oder gegebenenfalls eine oder zwei nicht benachbarte Methylengruppen durch -O-, -COO- und/oder -OOC-ersetzt sind und/oder gegebenenfalls eine Methylengruppe durch -CHX- ersetzt ist; und X Halogen, Cyano oder Methyl bedeutet.

2. Verbindungen nach Anspruch 1, gekennzeichnet durch die allgemeinen Formeln 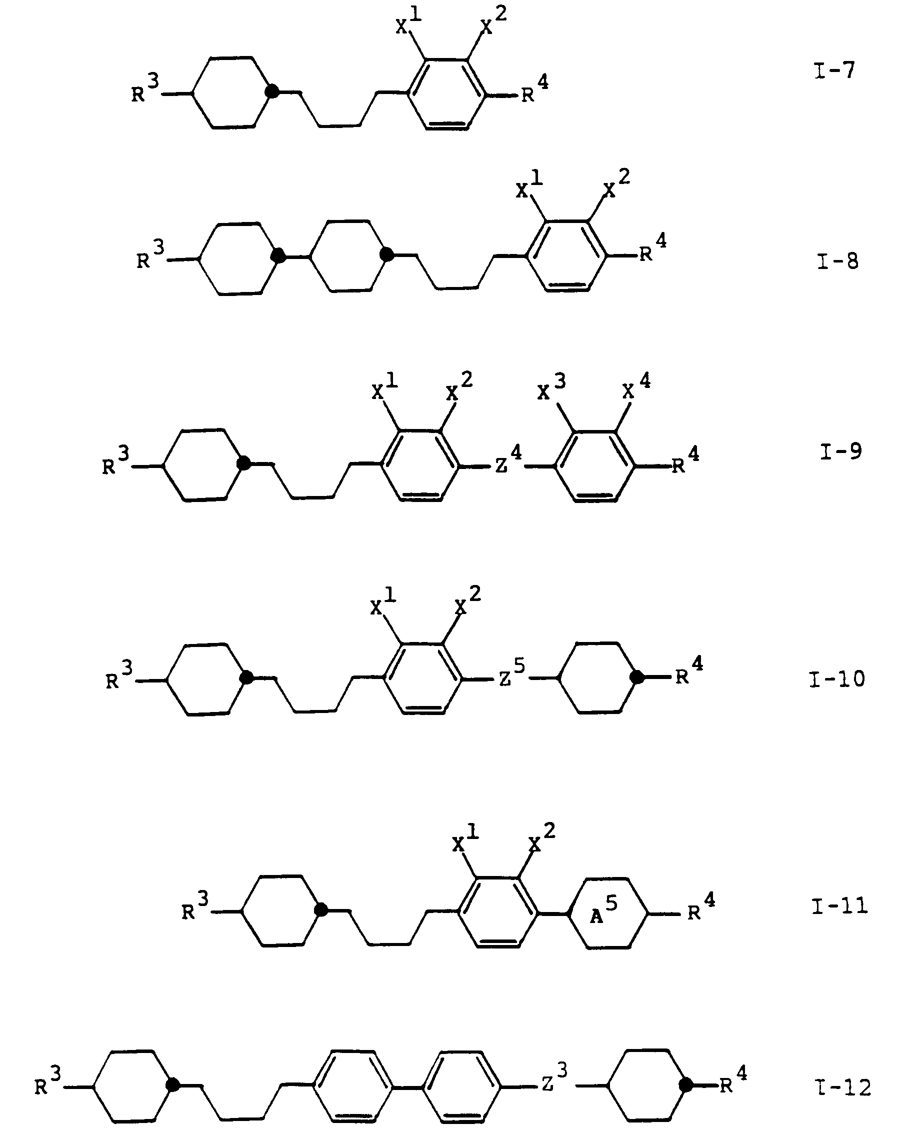 worin R³, R⁴ und Z³ die in Anspruch 1 gegebenen Bedeutungen haben; X¹, X², X³ und X⁴ unabhängig voneiander Wasserstoff oder Fluor bezeichnen; Z⁴ eine einfache Kovalenzbindung, -COO-, -OOC- oder -C≡C- bedeutet; Z⁵ eine einfache Kovalenzbindung, -OOC-, -OCH₂-, -CH₂CH₂-, -C≡C-, -O(CH₂)₃-, -(CH₂)₄- oder die trans-Form von -OCH₂-CH=CH-bezeichnet; und Ring A⁵ Pyridin-2,5-diyl, Pyrimdin-2,5-diyl, Pyrazin-2,5-diyl, trans-1,3-Dioxan-2,5-diyl, Bicyclo[2.2.2)octan-1,4-diyl, Naphthalin-2,6-diyl, Tetralin-2,6-diyl oder trans-Decalin-2,6-diyl darstellt.

3. Verbindungen nach Anspruch 1 oder 2, dadurch gekennzeichnet, dass R³ und R⁴ unabhängig voneinander Alkyl bezeichnen, in welchem gegebenenfalls eine >CH-CH< durch >C=C< ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch -CHX- ersetzt ist, oder R⁴ auch Halogen, Cyano, -NCS, -CF₃ oder -OCF₃ bezeichnet.

4. Verbindungen nach einem der Ansprüche 1 bis 3 dadurch gekennzeichnet, dass R³ und R⁴ höchstens je 18, vorzugsweise höchstens je 12 Kohlenstoffatome aufweisen.

5. Verbindungen nach einem der Ansprüche 1 bis 4, dadurch gekennzeichnet, dass R³ Alkyl, Alkenyl, Alkoxy, Alkenyloxy, Alkoxycarbonyl, Alkenyloxycarbonyl, Alkanoyloxy oder Alkenoyloxy, vorzugsweise Alkyl oder Alkenyl bedeutet.

6. Verbindungen nach einem der Ansprüche 1 bis 5, dadurch gekennzeichnet, dass R⁴ Alkyl, Alkenyl, Alkoxy, Alkenyloxy, Alkoxycarbonyl, Alkenyloxycarbonyl, Alkanoyloxy, Alkenoyloxy, Halogen, Cyano, -NCS, -CF₃ oder -OCF₃ bedeutet.

7. Flüssigkristallines Gemisch mit mindestens 2 Komponenten, dadurch gekennzeichnet, dass mindestens eine Komponente eine Verbindung der in Anspruch 1 definierten Formel I-1 ist.

8. Verwendung der Verbindungen der in Anspruch 1 definierten Formel I-1 für elektro-optische Zwecke.

## Claims

1. Compounds of the general formula 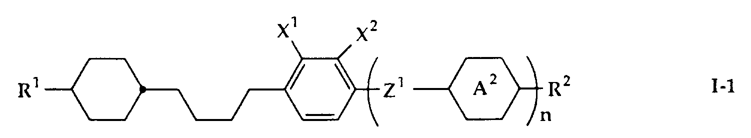 wherein n stands for the number 0 or 1; R¹ denotes a group R³ or R³-A³-Z²- and R² denotes a group R⁴ or R⁴-A⁴-Z³-; X¹ and X² each independently signify hydrogen or fluorine; A³ and A⁴ signify trans-1,4-cyclohexylene and ring A² signifies 1,4-phenylene, which is unsubstituted or mono- or disubstituted by fluorine, or trans-1,4-cyclohexylene, trans-1,3-dioxane-2,5-diyl, 1-cyano-trans-1,4-cyclohexylene, bicyclo[2.2.2]-octane-1,4-diyl, naphthalene-2,6-diyl, tetralin-2,6-diyl, trans-decalin-2,6-diyl pyridine-2,5-diyl, pyrimidine-2,5-diyl or pyrazine-2,5-diyl; Z¹, Z² and Z³ each signify a single covalent bond or one of groups Z¹, Z² and Z³ also signifies -COO-, -OOC-, -CH₂O-, -OCH₂-, -CH₂CH₂-, -C≡C-, -(CH₂)₃O-, -O(CH₂)₃-, -(CH₂)₄- or the trans form of -CH=CH-CH₂O- or -OCH₂-CH=CH-; R³ and R⁴ each independently denote halogen, cyano, -NCS, -CF₃, -OCF₃ or alkyl in which optionally one >CH-CH< is replaced by >C=C< and/or optionally one methylene group or two non-adjacent methylene groups is/are replaced by -O-, -COO- and/or -OOC- and/or optionally one methylene group is replaced by -CHX-; and X signifies halogen, cyano or methyl.

2. Compounds according to claim 1, characterized by the general formulae 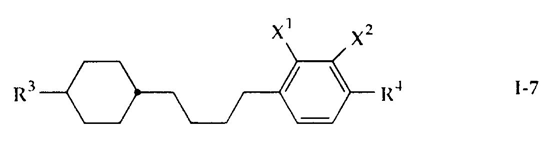 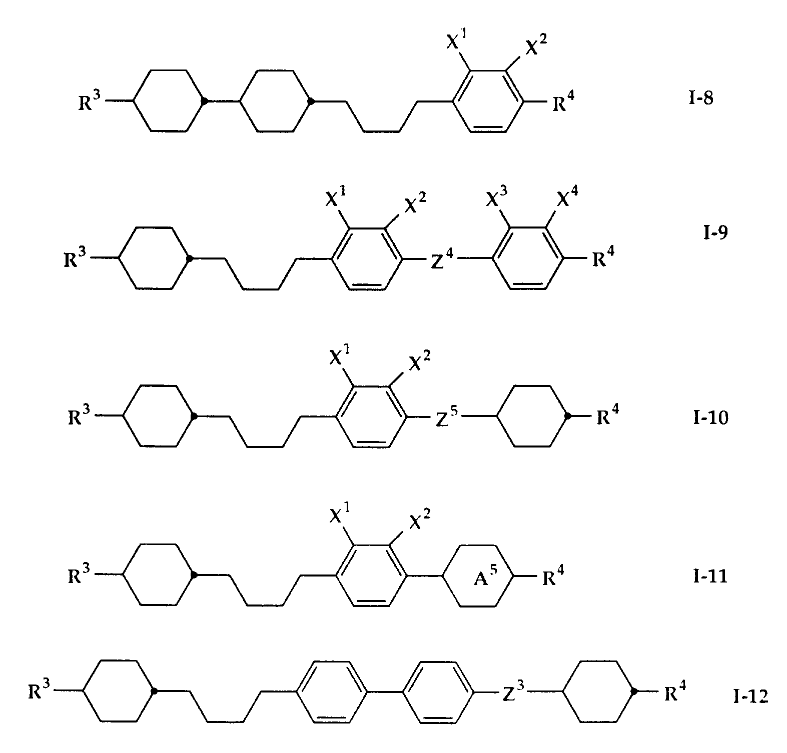 wherein R³, R⁴ and Z³ have the significances given in claim 1, X¹, X², X³ and X⁴ each independently denote hydrogen or fluorine, Z⁴ signifies a single covalent bond, -COO-, -OOC- or -C≡C-; Z⁵ denotes a single covalent bond, -OOC-, -OCH₂-, -CH₂CH₂-, -C≡C-, -O(CH₂)₃-, -(CH₂)₄- or the trans form of -OCH₂-CH=CH-; and ring A⁵ represents pyridine-2,5-diyl, pyrimidine-2,5-diyl, pyrazine-2,5-diyl, trans-1,3-dioxane-2,5-diyl, bicyclo[2.2.2]octane-1,4-diyl, naphthalene-2,6-diyl, tetralin-2,6-diyl or trans-decalin-2,6-diyl.

3. Compounds according to claim 1 or 2, characterized in that R³ and R⁴ each independently denote alkyl in which optionally one >CH-CH< is replaced by >C=C< and/or optionally one methylene group or two non-adjacent methylene groups is/are replaced by -O-, -COO- and/or -OOC- and/or optionally one methylene group is replaced by -CHX- or R⁴ also denotes halogen, cyano, -NCS, -CF₃ or -OCF₃.

4. Compounds according to any one of claims 1 to 3, characterized in that R³ and R⁴ each have a maximum of 18, preferably a maximum of 12, carbon atoms.

5. Compounds according to any one of claims 1 to 4, characterized in that R³ signifies alkyl, alkenyl, alkoxy, alkenyloxy, alkoxycarbonyl, alkenyloxycarbonyl, alkanoyloxy or alkenoyloxy, preferably alkyl or alkenyl.

6. Compounds according to any one of claims 1 to 5, characterized in that R⁴ signifies alkyl, alkenyl, alkoxy, alkenyloxy, alkoxycarbonyl, alkenyloxycarbonyl, alkanoyloxy, alkenoyloxy, halogen, cyano, -NCS, -CF₃ or -OCF₃.

7. A liquid crystalline mixture with at least 2 components, characterized in that at least one component is a compound of formula I-1 defined in claim 1.

8. The use of the compounds of formula I-1 defined in claim 1 for electro-optical purposes.

## Revendications

1. Composés de formule générale 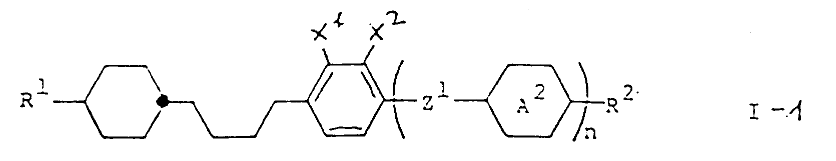 dans laquelle n représente le nombre 0 ou 1; R¹ représente un groupe R³ ou R³-A³-Z² et R² représente un groupe R⁴ ou R⁴-A⁴-Z²; X¹ et X² représentent indépendamment l'un de l'autre un hydrogène ou un fluor; A³ et A⁴ représentent un trans-1,4-cyclohexylène et le noyau A² représente un 1,4-phénylène non substitué ou mono- ou disubstitué par un fluor, ou un trans-1,4-cyclohexylène, trans-1,3-dioxanne-2,5-diyle, 1-cyano-trans-1,4-cyclohexylène, bicyclo[2.2.2]octane-1,4-diyle, naphtalène-2,6-diyle, tétralène-2,6-diyle ou trans-décaline-2,6-diyle, pyridine-2,5-diyle, pyrimidine-2,5-diyle ou pyrazine-2,5-diyle; Z¹, Z² et Z³ représentent chacun une liaison de covalence simple, ou l'un des groupes Z¹, Z² et Z³ représente également -COO-, -OOC-, -CH₂O-, -OCH₂-, -CH₂CH₂-, -C≡C-, -(CH₂)₃O-, -O(CH₂)₃-, -(CH₂)₄- ou la forme trans de -CH=CH-CH₂O-ou -OCH₂-CH=CH-; R³ et R⁴ représentent indépendamment l'un de l'autre un halogène, un cyano, -NCS, -CF₃, -OCF₃ ou un alkyle, où éventuellement un groupe >CH-CH< est remplacé par >C=C< et/ou éventuellement un ou deux groupes méthylène non voisins sont remplacés par -O-, -COO- et/ou -OOC- et/ou éventuellement un groupe méthylène est remplacé par -CHX-; et X représente un halogène, cyano ou méthyle.

2. Composés selon la revendication 1, caractérisés par les formules générales 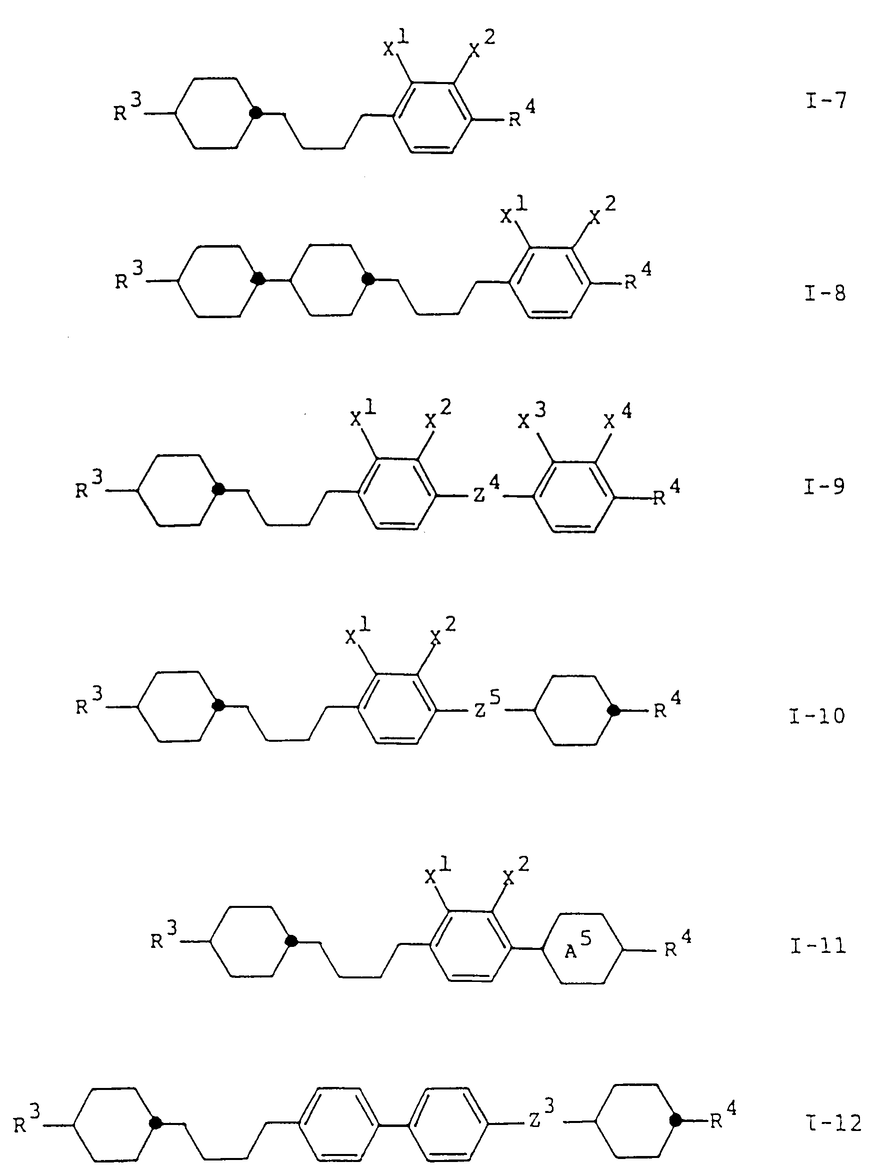 dans lesquelles R³, R⁴ et Z³ ont les significations données dans la revendication 1; X¹, X², X³ et X⁴ représentent indépendamment l'un de l'autre un hydrogène ou un fluor; Z⁴ représente une liaison de covalence simple, -COO-, -OOC- ou -C≡C-; Z⁵ représente une liaison de covalence simple, -OOC-, -OCH₂-, -CH₂CH₂-, -C≡C-, -O(CH₂)₃-, -(CH₂)₄- ou la forme trans de -OCH₂-CH=CH-; et le noyau A⁵ représente un pyridine-2,5-diyle, pyrimidine-2,5-diyle, pyrazine-2,5-diyle, trans-1,3-dioxanne-2,5-diyle, bicyclo[2.2.2]-octane-1,4-diyle, naphtalène-2,6-diyle, tétraline-2,6-diyle ou trans-décaline-2,6-diyle.

3. Composés selon la revendication 1 ou 2, caractérisés en ce que R³ et R⁴ représentent indépendamment l'un de l'autre un alkyle, où éventuellement un groupe >CH-CH< est remplacé par >C=C< et/ou éventuellement un ou deux groupes méthylène non voisins sont remplacés par -O-, -COO- et/ou -OOC- et/ou éventuellement un groupe méthylène est remplacé par -CHX-, ou R⁴ représente encore un halogène, un cyano, -NCS, -CF₃ ou -OCF₃.

4. Composés selon l'une des revendications 1 à 3, caractérisés en ce que R³ et R⁴ présentent au plus chacun 18, de préférence au plus chacun 12 atomes de carbone.

5. Composés selon l'une des revendications 1 à 4, caractérisés en ce que R³ représente un alkyle, alcényle, alcoxy, alcényloxy, alcoxycarbonyle, alcényloxycarbonyle, alcanoyloxy ou alcénoyloxy, de préférence un alkyle ou un alcényle.

6. Composés selon l'une des revendications 1 à 5, caractérisés en ce que R⁴ représente un alkyle, alcényle, alcoxy, alcényloxy, alcoxycarbonyle, alcényloxycarbonyle, alcanoyloxy, alcénoyloxy, halogène, cyano, -NCS, -CF₃ ou -OCF₃.

7. Mélange de cristaux liquides à au moins 2 composants, caractérisé en ce qu'au moins un composant est un composé de formule I définie dans la revendication 1.

8. Application des composés de formule I définie dans la revendication 1 à des buts électro-optiques.
